# EUROPEAN PATENT APPLICATION

(11) **EP 2 775 305 A1**
(43) Date of publication of application: **10.09.2014**
(21) Application number: 12846408.8
(22) Date of filing: 01.11.2012
(51) Int. Cl.: G01N 33/533, G01N 21/64, G01N 21/78, G01N 33/542

(54) **FLUOROIMMUNOASSAY METHOD USING POLYPEPTIDE COMPLEX CONTAINING FLUORO-LABELED ANTIBODY VARIABLE REGION**

(30) Priority: 02.11.2011 JP 2011241402
(71) Applicant: Ushio Denki Kabushiki Kaisha, Tokyo 100-8150 (JP)
(72) Inventor: UEDA, Hiroshi, Kanagawa 2268503 (JP); ABE, Ryoji, Yokohama-shi Kanagawa 225-0004 (JP); TAKAGI, Hiroaki, Yokohama-shi Kanagawa 225-0004 (JP)
(74) Representative: Wohlfahrt, Jan Günther
(86) International application number: PCT/JP2012/007025
(87) International publication number: WO 2013/065314

(57) **Abstract**

An object of the present invention is to provide an immunoassay that enables the rapid and convenient quantitative measurement with high detection sensitivity of a target substance in a liquid phase without the need of immobilization and washing steps and enables the visualization of an antigen. The present invention achieves the object by performing sequentially the steps of:
(a) bringing, in a liquid phase, a complex comprising a polypeptide containing an antibody light-chain variable region and a polypeptide containing an antibody heavy-chain variable region into contact with an antigen in a specimen for measurement, wherein either or both the polypeptides are labeled with a fluorescent dye(s);
(b) detecting the fluorescence of the fluorescent dye(s) or measuring the fluorescence intensity; and
(c) calculating the amount of the antigen contained in the sample or visualizing the antigen using the positive correlation between the antigen concentration and the fluorescence intensity of the fluorescent dye(s) as an indicator, thereby measuring the concentration of the target antigen existing in the test substance.

## Description

### Technical Field

The present invention relates to a kit for measuring and/or detecting the concentration of an antigen, and a method for measuring and/or detecting the concentration of an antigen, by which a low molecular weight compound can be detected with high sensitivity without the need of immobilization and washing steps.

### Background Art

Immunoassays using antibody-antigen binding are broadly employed for the detection of substances in specimens or the measurement of the concentrations thereof. The measurement method most broadly employed for clinical diagnosis, basic research, environmental research, and the like among these methods for measuring the concentrations of antigens and antibodies is a type of immunoassay referred to as a sandwich ELISA method (or sandwich RIA method). This method uses 2 types of monoclonal antibody that recognizes different epitopes of the same antigen, or a monoclonal antibody and a polyclonal antibody. The sandwich method is as specifically described below. The 1^{st} stage involves immobilizing a monoclonal or polyclonal antibody referred to as a primary antibody on a measurement plate, injecting a sample containing an antigen into the plate, and then allowing the plate to stand for a period of time for reaction so as to bind the antibody and the antigen. Next, the 2^{nd} stage involves removing contaminants bound to the antibody and any antigen that has nonspecifically bound to the plate by washing with a washing solution. The 3^{rd} stage involves injecting a solution of a labeled secondary antibody to which a reporter molecule such as an enzyme, a fluorescent dye, or a radioisotope has been bound in advance for a reaction to occur for a period of time, so that the labeled secondary antibody will bind to the antigen captured by the primary antibody. After this reaction, excess labeled antibody is removed by washing with a washing solution, the amount of the reporter molecule bound to the measurement plate is measured with the use of enzyme activity, fluorescence, a radioisotope, or the like, and thus the amount of the antigen in the sample is measured.

As described above, a general sandwich ELISA method requires two types of antibodies; the epitopes for which are different. When, for example, a low molecular weight compound or the like is used as an antigen, it is difficult to prepare a plurality of antibodies that recognize different epitopes. Accordingly, Ueda et al., have established a highly accurate immunoassay for low molecular weight compounds, which is referred to as an open sandwich method. This method uses a light chain variable region (VL) and a heavy chain variable region (VH) of a single antibody (Patent Documents 1 and 2, Non-patent Documents 1 and 2). This method is used to measure the concentration of an antigen and comprises preparing a VH-region polypeptide and a VL-region polypeptide of an antibody that specifically recognizes an antigen, labeling one of the polypeptides with a reporter molecule to prepare a labeled polypeptide, immobilizing the other polypeptide on a solid phase to prepare an immobilized polypeptide, bringing a specimen containing the antigen and the labeled polypeptide into contact with the immobilized polypeptide, and then measuring the amount of the reporter molecule of the labeled polypeptide bound to the immobilized polypeptide. Another example of a method for measuring a low molecular weight compound is a liquid chromatography method, in addition to immunoassays. However, such method is problematic in that it requires a highly accurate measuring instrument, a large amount of a test sample, and much time for measurement, and it has low general versatility.

Moreover, as immunoassays for measuring the concentration of an antigen using an antibody labeled with a fluorescent dye, an immunoassay that involves labeling an antibody and an antigen with different fluorescent dyes, and then using changes in fluorescence resonance energy transfer (FRET) efficiency taking place between the fluorescent dyes as indicators (Non-patent Documents 3 and 4), an immunoassay that involves using changes in efficiency due to quenching (and specifically, such method makes use of the phenomenon whereby the fluorescence of an antibody, which has been quenched by mixing a fluoro-labeled antibody in advance with a quenching substance, is increased through the introduction of a substance to be detected), and an immunoassay that involves measuring a decrease in fluorescence intensity resulting from the aggregation of an antibody (fluoro-labeled antibody) labeled with a fluorescent dye and a substance to be measured (Patent Document 3) are known.

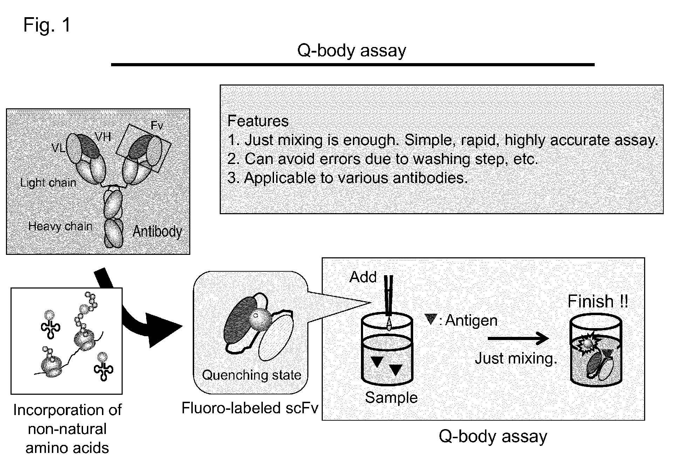

However, most immunoassays require a step of immobilizing an antibody or an antigen and a washing step for eliminating the adsorption of a non-specific labeling compound. Since these steps require complicated procedures, are time-consuming, and produce variable measurement results, the development of a liquid phase immunoassay that requires neither an immobilization step nor a washing step is required. Accordingly, the present inventors have developed "homogenous fluorescence immunoassay" (also referred to as a "homogenous fluorescent immunoassay method," "Quenchbody assay," or "Q-body assay") that is a liquid phase system requiring neither an immobilization step nor a washing step, enables the rapid and convenient quantitative measurement of a target substance, and allows visualization of an antigen (Patent Document 4, Non-patent Document 5, Figs. 1-3).

The above "homogenous fluorescence immunoassay" is a measurement method using technology that utilizes the quenching phenomenon, relating to:
(1) a kit for measuring and/or detecting the concentration of an antigen, which enables the measurement of the concentration of an antigen or the visualization of an antigen, using a positive correlation between the concentration of an antigen and the fluorescence intensity of a fluorescent dye in a liquid phase, as an indicator, wherein
   the kit is provided with an antibody light chain variable region (referred to as "VL") polypeptide and an antibody heavy chain variable region (referred to as "VH") polypeptide, and either the antibody light chain variable region polypeptide or the antibody heavy chain variable region polypeptide is labeled with a fluorescent dye; and
(2) the kit for measuring and/or detecting the concentration of an antigen according to (1), wherein the VL polypeptide and the VH polypeptide are bound to form a single-chain antibody. Specifically, this is a fluorescent immunoassay that comprises: mixing two antibody fragments in which either a VL polypeptide or a VH polypeptide is fluoro-labeled, or a single-chain antibody (scFv) in which a fluoro-labeled VL polypeptide and a VH polypeptide are bound to each other (with (1) and (2) being referred to as "Quenchbody" or "Q-body," (1) also being referred to as "VH+VL-type Q-body," and (2) also being referred to as "scFv-type Q-body") in a test specimen solution to be tested to determine the presence of an antigen; and enabling the measurement of the concentration of an antigen or the visualization of an antigen using a positive correlation between the fluorescence intensity of the fluorescent dye and the concentration of an antigen as an indicator (Figs. 1-3). The principle of such a measurement method is that highly conservative tryptophan residues within an antibody molecule interact with a fluorescent dye so as to quench the fluorescent dye, and the quenching is canceled in an antigen-dependent manner as a result of the addition of the antigen.

The "homogenous fluorescence immunoassay" technique using the Q-body is advantageous in that: 1) it is an extremely convenient measurement technique that requires no washing step, and complete measurement is possible with only the mixture of a small amount of a sample and the measurement of fluorescence intensity; 2) the quenching effect can be exhibited even when the antibody type is varied because of the use of highly conservative tryptophan residues existing in an antibody for quenching, and the technique has excellent general versatility such that it is broadly applicable to detection of various substances with the use of various antibodies; and 3) it is applicable in principle to a low molecular weight compound, since it requires only a single antigen site (see Patent Document 4, Fig. 1), for example. Moreover, the homogenous fluorescence immunoassay requires no washing step and is a convenient measurement method, and thus a measurement device therefor can be designed in a very compact size such that downsizing to a portable palm-sized device is possible. Therefore, even a general user who has never been professionally trained may be able to perform on-site measurements. Although this fluorescence immunoassay is very useful, as described above, the method is problematic in that the ratio of fluorescence intensity in the absence of an antigen to that in cases in which the antigen reaches a saturation point is as high as 1:6 and is as low as about 1:1.2. Hence, it has been expected that the dynamic range of measurement results would be extended, so as to increase the sensitivity and further improve the performance of the assay.

### Prior Art Document

### Patent Documents

Patent Document 1: JP Patent Publication (Kokai) No. H10-78436 A (1998)
Patent Document 2: JP Patent Number 3784111
Patent Document 3: JP Patent Publication (Kokai) No. H10 - 282098 A (1998)
Patent Document 4: WO2011/061944

### Non-patent Documents

Non-patent Document 1: Hiroshi Ueda, Yakugaku Zasshi 27: 71-80 (2007)
Non-patent Document 2: Lim SL, et al., Anal Chem. 79 (16): 6193-200 (2007)
Non-patent Document 3: Iijima 1. and Hohsaka T., Chembiochem. 17; 10 (6): 999-1006 (2009)
Non-patent Document 4: Kajihara D, et al., Nat Methods. 3 (11): 923 (2006)
Non-patent Document 5: Abe R, et al., J. Am. Chem. Soc. 133 (43): 17386-17394 (2011)

### Summary of the Invention

### Problem to Be Solved by the Invention

An objective of the present invention is to provide an immunoassay, which:
enables rapid and convenient detection and/or quantitative measurement of a target substance in a liquid phase without the need for immobilization and washing steps, and allows visualization of an antigen; and
is a fluorescence immunoassay that exhibits an even wider dynamic range for measurement results and high sensitivity.

### Means for Solving the Problem

A fluoro-labeled single-chain antibody (scFv) that had been quenched in an antigen-free solution and then denatured with guanidine hydrochloride had almost the same fluorescence intensity as that in a case in which the antigen reached a saturation point. Based on this, the present inventors considered that increasing the quenching efficiency in the absence of an antigen would be an effective means for increasing the dynamic range. Thus, the following examination was performed.

Specifically, the present inventors speculated that:
quenching might mainly result from contact of tryptophan residues highly conserved in a VL polypeptide and a VH polypeptide with a fluorescent dye(s) used for labeling; and
such fluorescent dye(s) might be moved out from an antigen-binding pocket as a result of stabilization of the structure of a variable region accompanying antigen binding, thereby canceling the quenching state and increasing fluorescence intensity.

It was predicted that a VL polypeptide and a VH polypeptide existing as 2 different types of protein would result in lower contact efficiency between fluorescent dye(s) and tryptophan residues and lower the quenching level because of dissociation and weak interaction between the VL polypeptide and the VH polypeptide. Also, in the case of a single-chain antibody (scFv), the interaction between a VL polypeptide and a VH polypeptide can be increased through linking of the VL polypeptide and the VH polypeptide with an artificial peptide linker to form a single-chain antibody. However, it was thought that this could decrease original antibody functions such as its activity to bind to an antigen and its stability because of the addition of such an artificial peptide linker. The present inventors speculated that a Fab fragment might retain its original antibody functions, if a polypeptide comprising an antibody light chain variable region (VL) and an antibody light chain constant domain and a polypeptide comprising an antibody heavy chain variable region (VH) and an antibody heavy chain constant domain form the Fab (Fragment, antigen binding) comprising 1 molecule of a hetero dimer protein wherein such polypeptides are bound via a disulfide bond. The present inventors discovered that when either a VH-containing polypeptide or a VL-containing polypeptide of a Fab fragment was fluoro-labeled, the fluorescent dye was more strongly quenched in the absence of the antigen so as to allow lowering of the background. Moreover, the present inventors discovered that when a VH-containing polypeptide and a VL-containing polypeptide of a Fab fragment were each labeled with fluorescent dyes of the same color, a quenching effect (H-dimer) due to the interaction between dyes was obtained in addition to quenching due to contact between the fluorescent dyes and tryptophan residues, and thus higher detection sensitivity could be obtained. The present inventors discovered that when a VH-containing polypeptide and a VL-containing polypeptide of a Fab fragment were each further labeled with fluorescent dyes of different colors, a quenching effect resulting from the FRET effect was obtained in addition to quenching due to the interaction between fluorescent dyes and tryptophan residues and the quenching effect due to contact between dyes, and thus even higher detection sensitivity could be obtained. Furthermore, the present inventors discovered that when a VH-containing polypeptide and a VL-containing polypeptide of a Fab fragment were labeled with a fluorescent dye and a quencher for quenching the fluorescent dye, respectively, quenching due to the interaction between the fluorescent dye and tryptophan residues and the quenching effect between the fluorescent dye and the quencher, and thus high detection sensitivity, could be obtained.

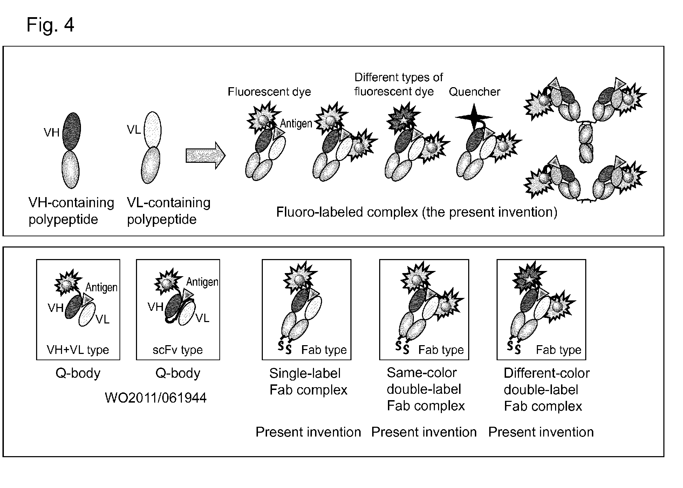

The present inventors further discovered that when the thermal stability of a conventional fluoro-labeled single-chain antibody (scFv) and the same of the fluoro-labeled Fab complex of the present invention were measured, the fluoro-labeled Fab complex was excellent in terms of heat resistance and preservation such that it was denatured at 73°C, although the fluoro-labeled single-chain antibody (scFv) was denatured at 61°C. The present invention was completed based on these findings (Fig. 4).

Specifically, the present invention relates to:
[1] A kit for measuring and/or detecting the concentration of an antigen, which is characterized by enabling the measurement of the concentration of an antigen or the visualization of an antigen using a positive correlation between the concentration of an antigen and the fluorescence intensity of a fluorescent dye in a liquid phase, as an indicator, wherein,
   either or both a polypeptide containing an antibody light chain variable region and a polypeptide containing an antibody heavy chain variable region are labeled with a fluorescent dye, and
   the kit is provided with a complex comprising the polypeptide containing the antibody light chain variable region and the polypeptide containing the antibody heavy chain variable region;
[2] The kit for measuring and/or detecting the concentration of an antigen according to [1] above, which is characterized in that the polypeptide containing the antibody light chain variable region and the polypeptide containing the antibody heavy chain variable region are each labeled with the same fluorescent dye;
[3] The kit for measuring and/or detecting the concentration of an antigen according to [1] above, which is characterized in that the polypeptide containing the antibody light chain variable region and the polypeptide containing the antibody heavy chain variable region are each labeled with a different type of fluorescent dye;
[4] The kit for measuring and/or detecting the concentration of an antigen according to [1] above, which is characterized in that either the polypeptide containing the antibody light chain variable region or the polypeptide containing the antibody heavy chain variable region is labeled with a fluorescent dye and the other is labeled with a quencher for quenching the fluorescent dye;
[5] The kit for measuring and/or detecting the concentration of an antigen according to [1] above, which is characterized in that either the polypeptide containing the antibody light chain variable region or the polypeptide containing the antibody heavy chain variable region is labeled with a fluorescent dye;
[6] The kit for measuring and/or detecting the concentration of an antigen according to any one of [1] to [5] above, which is characterized in that a complex comprising the polypeptide containing the antibody light chain variable region and the polypeptide containing the antibody heavy chain variable region is a Fab (Fragment, antigen binding);
[7] The kit for measuring and/or detecting the concentration of an antigen according to any one of [1] to [6] above, which is characterized in that the fluorescent dye is selected from a rhodamine-based fluorescent dye and an oxazine-based fluorescent dye;
[8] The kit for measuring and/or detecting the concentration of an antigen according to [7] above, which is characterized in that the fluorescent dye is selected from carboxy rhodamine 110, carboxytetramethyl rhodamine, and ATTO 655 (trade name); and
[9] The kit for measuring and/or detecting the concentration of an antigen according to any one of [4] to [8] above, which is characterized in that the quencher is 7-nitrobenzofurazan (NBD).

The present invention further relates to:
[10] A method for measuring and/or detecting the concentration of an antigen, which is characterized by comprising the following steps (a) to (c) in sequence,
   (a) a step of bringing a complex into contact with an antigen in a specimen for measurement, wherein
      the complex comprises a polypeptide containing an antibody light chain variable region and a polypeptide containing an antibody heavy chain variable region, in which either or both polypeptides are labeled with a fluorescent dye;
   (b) a step of detecting the fluorescence of the fluorescent dye, or measuring the fluorescence intensity of the fluorescent dye; and
   (c) a step of calculating the amount of the antigen contained in a sample or visualizing the antigen, using a positive correlation between the concentration of the antigen and the fluorescence intensity of the fluorescent dye as an indicator;
[11] The method for measuring and/or detecting the concentration of an antigen according to [10] above, which is characterized in that the polypeptide containing the antibody light chain variable region and the polypeptide containing the antibody heavy chain variable region are each labeled with the same fluorescent dye;
[12] The method for measuring and/or detecting the concentration of an antigen according to [10] above, which is characterized in that the polypeptide containing the antibody light chain variable region and the polypeptide containing the antibody heavy chain variable region are each labeled with a different type of fluorescent dye;
[13] The method for measuring and/or detecting the concentration of an antigen according to [10] above, which is characterized in that either the polypeptide containing the antibody light chain variable region or the polypeptide containing the antibody heavy chain variable region is labeled with a fluorescent dye, and the other is labeled with a quencher for quenching the fluorescent dye;
[14] The method for measuring and/or detecting the concentration of an antigen according to [10] above, which is characterized in that either the polypeptide containing the antibody light chain variable region or the polypeptide containing the antibody heavy chain variable region is labeled with a fluorescent dye;
[15] The method for measuring and/or detecting the concentration of an antigen according to any one of [10] to [14] above, which is characterized in that the complex comprising the polypeptide that contains the antibody light chain variable region and the polypeptide that contains the antibody heavy chain variable region is a Fab (Fragment, antigen binding);
[16] The method for measuring and/or detecting the concentration of an antigen according to any one of [10] to [15] above, which is characterized in that the antigen is a low molecular weight compound; and
[17] The method for measuring and/or detecting the concentration of an antigen according to any one of [10] to [15] above, which is characterized in that the antigen is human osteocalcin, bisphenol A, serum albumin, clenbuterol, ractopamine, cotinine, influenza A virus hemagglutinin, a morphine, a methamphetamine, cocaine, tetrahydrocannabinol, or ketamine.

### Effect of the Invention

According to the present invention, a target substance can be detected and/or quantitatively measured rapidly and conveniently in a liquid-phase system. Moreover, a highly-sensitive immunoassay capable of measuring a low molecular weight compound and a kit for measuring an antigen by this immunoassay can be provided. The measurement method of the present invention enables detection and/or measurement of the binding of an antigen with a complex (hereinafter, also referred to as the "fluoro-labeled complex of the present invention") comprising a polypeptide (hereinafter, also referred to as "VL-containing polypeptide") that contains an antibody light chain variable region and a polypeptide (hereinafter, also referred to as "VH-containing polypeptide") that contains an antibody heavy chain variable region, wherein either or both polypeptides are labeled with a fluorescent dye(s), with the use of the fluorescence intensity of the fluorescent dye as an indicator. Since the above fluorescent dye(s) is in an effectively quenched state when the fluoro-labeled complex of the present invention is not bound to an antigen, the antigen can be detected and/or measured with good sensitivity.

### Brief Description of the Drawings

Fig. 1 shows the characteristics of Q-body assay. The concentration of an antigen can be rapidly measured with high sensitivity only by mixing an antigen with 2 types of polypeptide (VH+VL), an antibody heavy chain variable region polypeptide and an antibody light chain variable region polypeptide, one of which is fluoro-labeled, or, a fluoro-labeled single-chain antibody (scFv) prepared by linking an antibody heavy chain variable region polypeptide and an antibody light chain variable region polypeptide (see WO2011/061944).

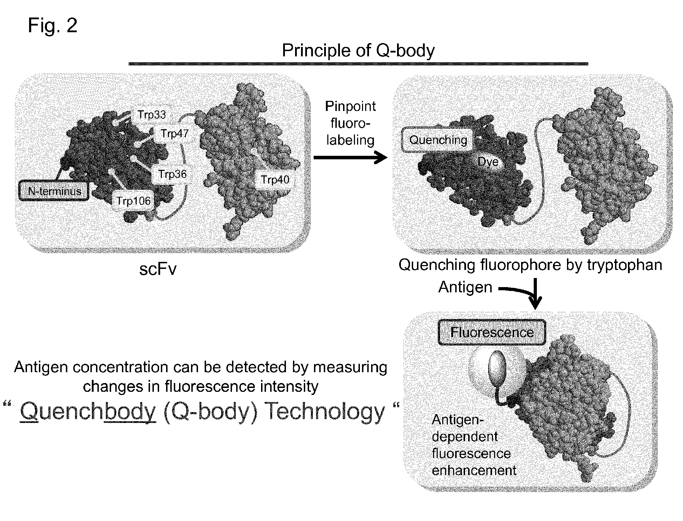

Fig. 2 shows the principle of Q-body. Fluorescence is quenched in the absence of an antigen due to the interaction between fluorescent dyes and amino acids that are broadly conserved in an antibody variable region, specifically Trp 33, Trp 47, Trp 36, and Trp 106 of the antibody heavy chain variable region and Trp 40 of the antibody light chain variable region. However, quenching is canceled in an antigen concentration-dependent manner and thus fluorescence intensity increases. Such fluoro-labeled (VH+VL) and fluoro-labeled scFv are referred to as "Quench body (Q-body)."

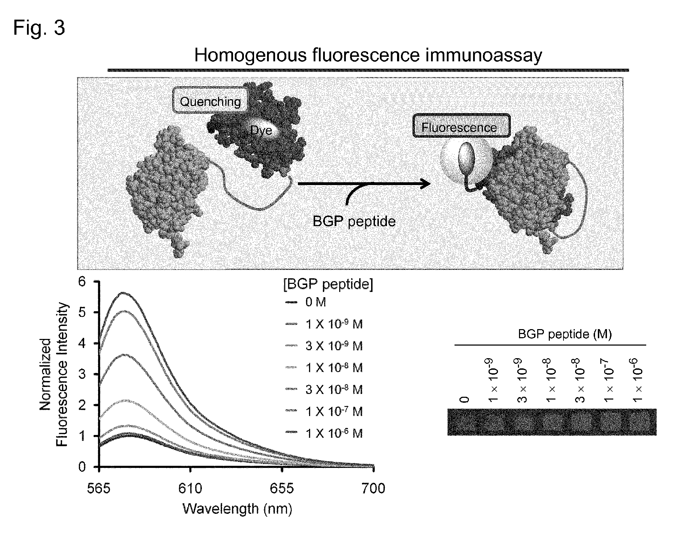

Fig. 3 shows an example of the implementation of a homogenous fluorescence immunoassay whereby antigen concentration was measured using Q-body. TAMRA-labeled anti-BGP single-chain antibody (scFv) was used as Q-body and antigen concentration was varied. Fig. 3 shows the results of measuring fluorescence emission spectrum using a spectrophotofluorometer (FluoroMax-4), and fluorescence intensity using a fluorescent image analyzer (FM-BIOIII).
Fig. 4 schematically shows a complex comprising a polypeptide that contains an antibody light chain variable region and a polypeptide that contains an antibody heavy chain variable region, either or both of which are labeled with a fluorescent dye. Specifically, Fig. 4 schematically shows the fluoro-labeled complex of the present invention (upper figure). Fig. 4 also schematically shows Q-body used in Examples, and, the single-label Fab complex, the same-color double-label Fab complex, and the different-color double-label Fab complex of the present invention (lower figure).

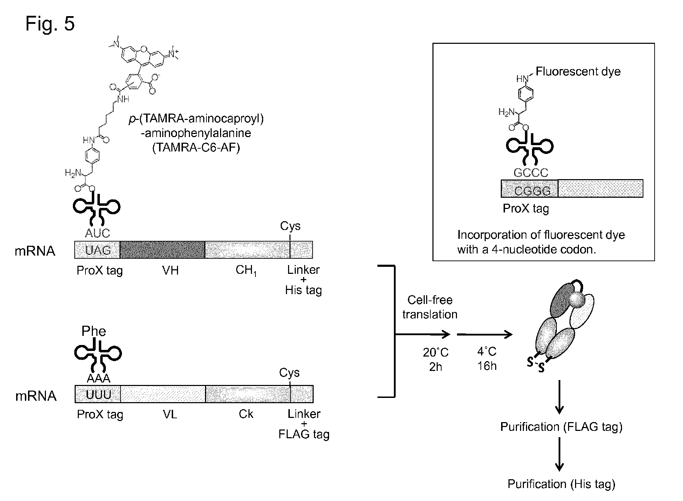

Fig. 5 schematically shows a method for preparing the fluoro-labeled Fab complex of the present invention. The single-label Fab complex of the present invention was prepared as follows. A plasmid containing ProX tag (TAG), VH, CH₁, and a gene prepared by adding the DNA sequences of a linker and an His tag to the C terminus, a plasmid containing ProX tag (TTT), VL, Cκ, and a gene prepared by adding the DNA sequences of a linker and a FLAG tag to the C terminus, and TAMRA-AF-tRNA amber (CloverDirect) were added to an E. *coli* cell-free synthesis kit (Remarkable Yield Translation System Kit (RYTS)), followed by 2 hours of reaction at 20°C, and then the TAMRA-labeled VH-containing polypeptide and VL-containing polypeptide were synthesized via co-expression. Thereafter, the resultant was left to stand at 4°C for 16 hours, so as to form a complex. Protein purification was performed using FLAG and His tags added to the C terminus. A same-color double-label Fab complex was synthesized and purified by a method similar to that for the single-label Fab complex using a plasmid constructed by adding a ProX tag (TAG) to the N terminus of the VH-containing gene and the VL-containing gene. A different-color double-label Fab complex was synthesized and purified by the above method that involves using a plasmid constructed by adding a ProX tag (TAG) and a ProX tag (CGGG) to the N terminus of one of the genes, and adding an A-AF-tRNA amber dye and a B-AF-tRNA CGGG dye (CloverDirect). A schematic diagram showing the incorporation of fluoro-labeled amino acids with a four-base codon is shown within the framework.

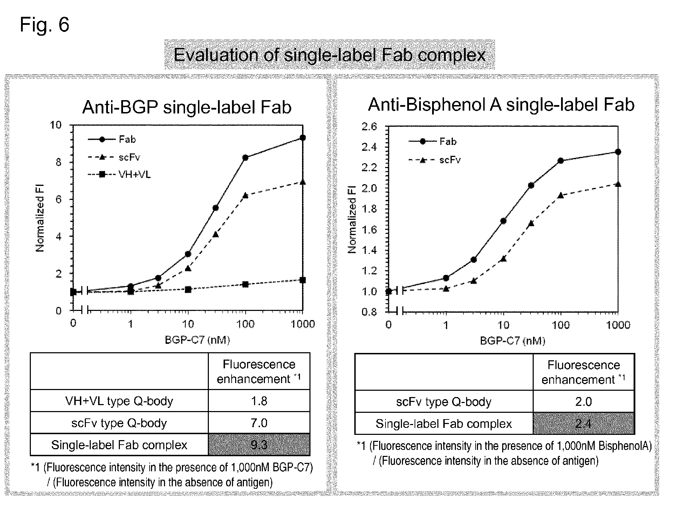

Fig. 6 shows that BGP and bisphenol A could be measured using the single-label Fab complex of the present invention with a fluorescence intensity ratio higher than those of VH+VL-type Q-body and scFv-type Q-body.

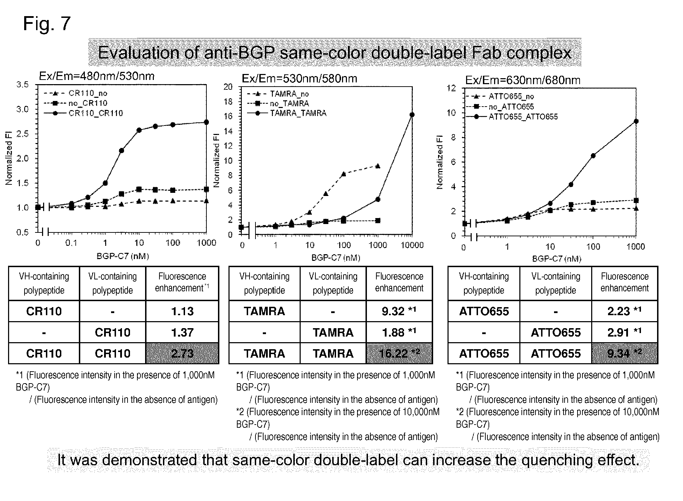

Fig. 7 shows that BGP could be measured using the same-color double-label Fab complex of the present invention with a fluorescence intensity ratio higher than that of the single-label Fab complex of the present invention.

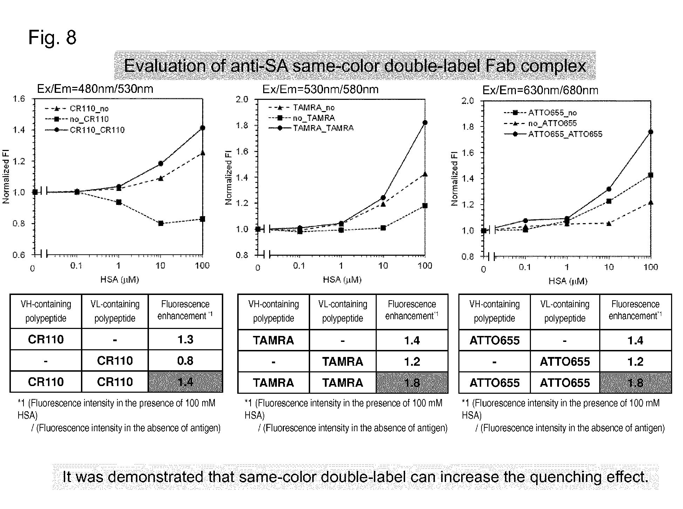

Fig. 8 shows that HSA could be measured using the same-color double-label Fab complex of the present invention with a fluorescence intensity ratio higher than that of the single-label Fab complex of the present invention.

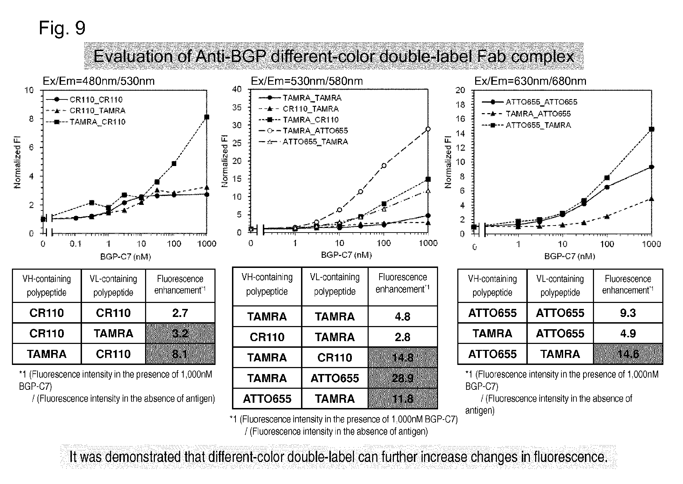

Fig. 9 shows that BGP could be measured using the different-color double-label Fab complex of the present invention with a fluorescence intensity ratio higher than that of the single-label Fab complex of the present invention.

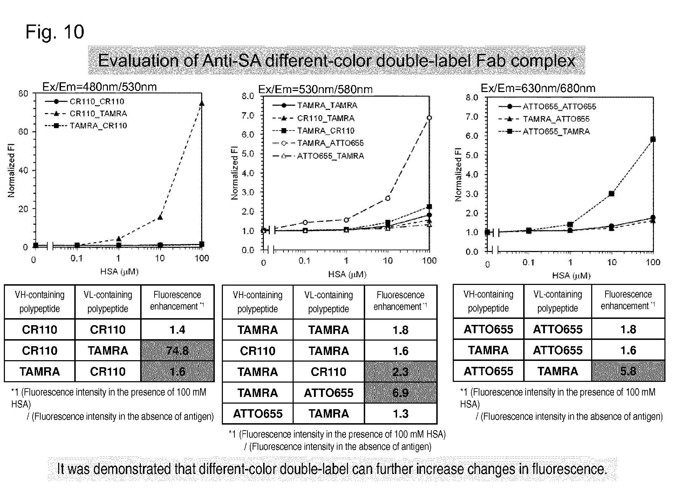

Fig. 10 shows that HSA could be measured using the different-color double-label Fab complex of the present invention with a fluorescence intensity ratio higher than that of the single-label Fab complex of the present invention.

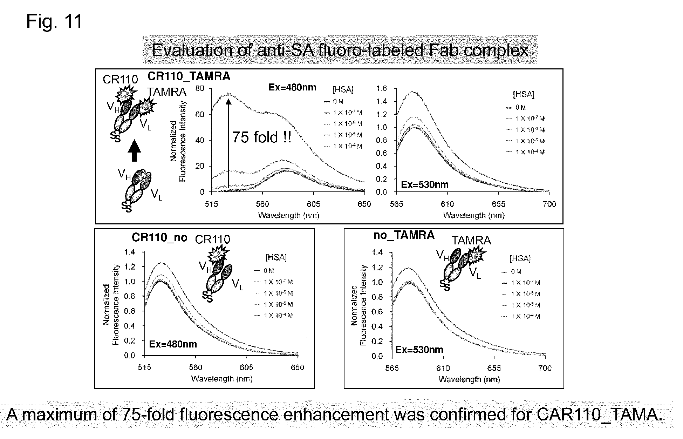

Fig. 11 shows that HSA could be measured using a different-color double-label Fab complex of an anti-SA antibody, which comprises a polypeptide containing a CR110-labeled anti-SA antibody heavy chain variable region (VH) and an antibody heavy chain constant domain (CH₁) and a polypeptide containing a TAMRA-labeled anti-SA antibody light chain variable region (VL) and an antibody light chain constant domain (Cκ), with a fluorescence intensity ratio higher than that of the single-label Fab complex of the present invention.

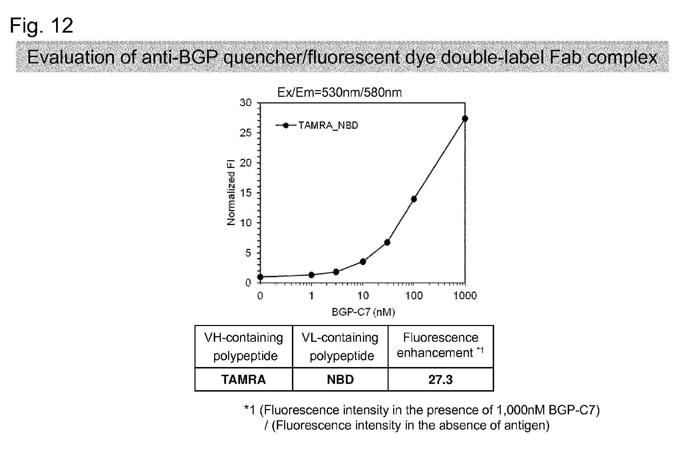

Fig. 12 shows that BGP could be measured using the Fab complex of the present invention comprising a polypeptide labeled with a fluorescent dye and the other polypeptide labeled with a quencher for quenching the fluorescent dye, with a high fluorescence intensity ratio. Measurement was always performed with Ex/Em= 530/580.

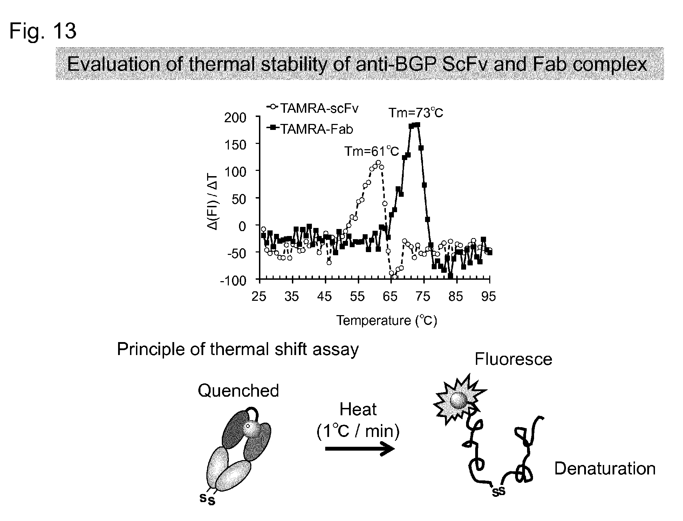

Fig. 13 shows that the fluoro-labeled Fab complex of the present invention is excellent in thermostability such that the temperature at which the Fab complex was thermally denatured was higher than the temperature at which the fluoro-labeled scFv was thermally denatured, by 12°C.

### Modes for Carrying Out the Invention

The kit for measuring and/or detecting the concentration of an antigen of the present invention is not particularly limited, as long as it is a kit for measuring and/or detecting the concentration of an antigen, which is characterized in that:
the kit is provided with a complex that comprises a polypeptide (VL-containing polypeptide) containing an antibody light chain variable region and a polypeptide (VH-containing polypeptide) containing an antibody heavy chain variable region, wherein either or both the VL-containing polypeptide and the VH-containing polypeptide are labeled with a fluorescent dye(s); and
the kit enables the measurement of the concentration of an antigen or the visualization of an antigen using a positive correlation between the concentration of the antigen and the fluorescence intensity of the fluorescent dye in a liquid phase, as an indicator. The kit may also be provided with an antigen that can be used as a standard substance, reagents, tools, instruction manuals, and the like that are generally used for this type of immunoassay kit, in addition to contain as a component the above complex comprising a VL-containing polypeptide and a VH-containing polypeptide, one of or both of which are labeled with a fluorescent dye(s).

The above antigen is not particularly limited, as long as it is an antigen that is specifically recognized by the above VH-containing polypeptide, the above VL-containing polypeptide, or a complex comprising these polypeptides. Examples thereof include a protein, a peptide, a carbohydrate, a lipid, a glycolipid, and a low molecular weight compound, as well as proteins subjected to protein modification such as phosphorylation or methylation. The kit for measuring and/or detecting the concentration of an antigen of the present invention is excellent in detection sensitivity, and thus is particularly useful for detection of a low molecular weight compound.

In the kit for measuring and/or detecting the concentration of an antigen of the present invention, either or both a VL-containing polypeptide and a VH-containing polypeptide that compose a complex may be labeled with a fluorescent dye(s). Specifically, the complex that may be used herein is (i) a complex in which either the VL-containing polypeptide or the VH-containing polypeptide is labeled with a fluorescent dye, (ii) a complex in which these polypeptides are labeled with the same fluorescent dye, (iii) a complex in which these polypeptides are labeled with different types of fluorescent dye, or (iv) a complex in which one of these polypeptides is labeled with a fluorescent dye and the other polypeptide is labeled with a quencher for quenching the fluorescent dye. When added to either a VH-containing polypeptide or a VL-containing polypeptide, a fluorescent dye may be added to any one of these polypeptides, and is preferably added to the one so that high detection sensitivity can be obtained. When two different types of fluorescent dye are added to a VH-containing polypeptide and a VL-containing polypeptide, respectively, the combination of a polypeptide and a fluorescent dye to be added thereto is not limited, and is preferably a combination of a fluorescent dye and a polypeptide by which high detection sensitivity can be obtained. A VL-containing polypeptide and a VH-containing polypeptide may be: labeled with a protein comprising an arbitrary amino acid sequence, a peptide tag such as a ProX tag (SEQ ID NO: 1), a FLAG tag, a His tag, an HA tag, or an Ni tag, a linker comprising an arbitrary amino acid sequence, a stable radio isotope, an enzyme, or a fluorescent dye that is a type differing from that of the above fluorescent dye; and may further be subjected to modification such as sugar chain addition, phosphorylation, and methylation, as long as the light emission, detection, and quenching of the above fluorescent dye are not inhibited.

The antibody light chain variable region (VL) is not particularly limited, as long as it contains an amino acid sequence specific to an antibody light chain variable region (VL) encoded by an exon of the V and J regions of an antibody light chain gene. The antibody light chain variable region (VL) may be prepared by adding an arbitrary amino acid sequence to the N-terminus and/or the C-terminus of an amino acid sequence specific to the above antibody light chain variable region, or prepared by deleting, substituting, or inserting 1, 2 or more amino acids, as long as the affinity of the above VL-containing polypeptide or the fluoro-labeled complex of the present invention for an antigen is not negatively affected. The affinity for an antigen can be adequately examined by a conventional method such as ELISA or FACS. Moreover, an amino acid sequence specific to the above antibody light chain variable region is preferably an amino acid sequence in which the 35^{th} amino acid (as numbered using the Kabat numbering system) is tryptophan.

The antibody heavy chain variable region (VH) is not particularly limited, as long as it contains an amino acid sequence specific to an antibody heavy chain variable region (VH) encoded by an exon of the V, D, and J regions of an antibody heavy chain gene. The antibody heavy chain variable region (VH) may be prepared by adding an arbitrary amino acid sequence to the N-terminus and/or the C-terminus of an amino acid sequence specific to the above antibody heavy chain variable region, or prepared by deleting, substituting, or inserting 1, 2 or more amino acids, as long as the affinity of the above VH-containing polypeptide or the fluoro-labeled complex of the present invention for an antigen is not negatively affected. The affinity for an antigen can be adequately examined by a conventional method such as ELISA or FACS. Moreover, an amino acid sequence specific to the above antibody heavy chain variable region is preferably an amino acid sequence in which the 36^{th}, the 47^{th}, or 103^{rd} amino acid (as numbered using the Kabat numbering system) is tryptophan.

Any VL-containing polypeptide may be used herein as long as it contains an antibody light chain variable region (VL), and it can contain an antibody light chain or a peptide comprising an arbitrary amino acid sequence in an antibody light chain. For example, the VL-containing polypeptide may be prepared by adding an antibody light chain constant domain (Cκ) and a hinge portion to an antibody light chain variable region (VL), and is particularly preferably a polypeptide prepared by adding Cκ to VL, for example. A specific example of the above VL-containing polypeptide is preferably a polypeptide comprising an amino acid sequence prepared by adding SEQ ID NO: 4 to SEQ ID NO: 5, SEQ ID NO: 4 to SEQ ID NO: 7, SEQ ID NO: 4 to SEQ ID NO: 10, SEQ ID NO: 4 to SEQ ID NO: 15, SEQ ID NO: 4 to SEQ ID NO: 17, SEQ ID NO: 4 to SEQ ID NO: 19, SEQ ID NO: 4 to SEQ ID NO: 21, SEQ ID NO: 4 to SEQ ID NO: 23, SEQ ID NO: 4 to SEQ ID NO: 25, SEQ ID NO: 4 to SEQ ID NO: 27, or SEQ ID NO: 4 to SEQ ID NO: 29. Furthermore, a VL-containing polypeptide capable of recognizing an antigen can be adequately prepared depending on the antigen to be measured.

Any VH-containing polypeptide may be used herein, as long as it contains an antibody heavy chain variable region (VH), and it can contain an antibody heavy chain or a peptide comprising an arbitrary amino acid sequence in the antibody heavy chain. For example, the VH-containing polypeptide may be prepared by adding an antibody heavy chain constant domain (CH₁), and further a hinge portion and an Fc region to an antibody heavy chain variable region (VH), and is particularly preferably a polypeptide prepared by adding CH₁ to VH, for example. A specific example of the above VH-containing polypeptide is preferably a polypeptide comprising an amino acid sequence prepared by adding SEQ ID NO: 6 to SEQ ID NO: 3, SEQ ID NO: 6 to SEQ ID NO: 9, SEQ ID NO: 6 to SEQ ID NO: 12, SEQ ID NO: 6 to SEQ ID NO: 16, SEQ ID NO: 6 to SEQ ID NO: 18, SEQ ID NO: 6 to SEQ ID NO: 20, SEQ ID NO: 6 to SEQ ID NO: 22, SEQ ID NO: 6 to SEQ ID NO: 24, SEQ ID NO: 6 to SEQ ID NO: 26, SEQ ID NO: 6 to SEQ ID NO: 28, or SEQ ID NO: 6 to SEQ ID NO: 30. Furthermore, a VH-containing polypeptide capable of recognizing an antigen can be adequately prepared depending on the antigen to be measured.

The VL-containing polypeptide and the VH-containing polypeptide preferably form a complex, and are not particularly limited, as long as peptides containing amino acid sequences that form the complex are bound to an antibody light chain variable region (VL) and an antibody heavy chain variable region (VH), respectively. Examples of peptides that form a complex include the above antibody constant domains (e.g., CH₁ and Cκ). Moreover, one of the peptides forming a dimer can be added to VL and the other can be added to VH. Furthermore, two types of proteins, which interact with each other to contribute to the formation of such a complex, can also be selected.

The "complex" of the fluoro-labeled complex of the present invention may be any complex, as long as it contains a VL-containing polypeptide and a VH-containing polypeptide as components that form the complex. The complex may further contain as components, a peptide, a protein, a lipid, a metal, and other compounds, for example, in addition to the above VL-containing polypeptide and VH-containing polypeptide, as long as the functions of the fluoro-labeled complex of the present invention are not impaired.

Furthermore, the complex of the present invention may be any structure such that the above polypeptides are combined to be able to function integrally. In this case, the presence or the absence of a chemical bond between the above polypeptides is a matter of no importance. Examples of such a bond include a disulfide bond between the above polypeptides and a bond formed using a cross-linking agent. These (plurality of) bonds may be used in combination in one complex. In particular, a preferable example thereof is a disulfide bond. The complex of the present invention is preferably formed of the above polypeptides located close to each other, or comprises a VL-containing polypeptide and a VH-containing polypeptide that contain peptides having such functions. An antibody light chain constant domain and an antibody heavy chain constant domain in an antibody molecule interact with each other so that the antibody light chain variable region and the antibody heavy chain variable region are located closer to each other, thereby serving an ancillary role to form a strong antigen-binding pocket. Accordingly, as the complex of the present invention, a fragment antigen-binding (Fab) fragment that is composed of two polypeptides, which comprises one variable region and one constant domain of each of a light chain and a heavy chain of an antibody and each of the polypeptides is bound via a disulfide bond, a F(ab')₂ fragment wherein two Fab fragments are disulfide-bonded via a hinge, or a full-length antibody is preferred. In particular, a Fab fragment is most preferred. Such fluoro-labeled complex of the present invention that forms a Fab fragment comprising a VL-containing polypeptide and a VH-containing polypeptide may also be referred to as "the fluoro-labeled Fab complex of the present invention." In particular, the fluoro-labeled Fab complex of the present invention in which either a VL-containing polypeptide or a VH-containing polypeptide is fluoro-labeled may also be referred to as "the single-label Fab complex of the present invention." Furthermore, the fluoro-labeled Fab complex of the present invention in which both a VL-containing polypeptide and a VH-containing polypeptide are fluoro-labeled may also be referred to as "the same-color double-label Fab complex of the present invention" when the two types of fluorescent dye are the same or referred to as "the different-color double-label Fab complex of the present invention" when the two types of fluorescent dye are different.

In the present invention, a VL-containing polypeptide, a VH-containing polypeptide, a complex containing these polypeptides, its components, and the like can be prepared by known chemical synthesis methods, gene recombination techniques, methods for the denaturation of an antibody molecule using protease, and the like. In particular, they can be preferably prepared by gene recombination techniques by which mass preparation is possible with relatively simple operation. When the above polypeptides are prepared by gene recombination techniques, DNA containing the nucleotide sequence encoding such a polypeptide is introduced into an appropriate expression vector to construct a recombinant vector, and then a target polypeptide can be expressed using an expression system using bacterial, yeast, insect, animal/plant cells, or the like as host cells or a cell-free translation system (Fig. 5). When a target polypeptide is expressed in a cell-free translation system, for example, the target polypeptide can be expressed in a reaction solution prepared by adding nucleotide triphosphate and various amino acids to a cell-free extract of such as *Escherichia coli,* wheat germ, or rabbit reticulocytes. At this time, a tag such as a ProX tag, a FLAG tag, or a His tag may be added to a VL-containing polypeptide and a VH-containing polypeptide. These tags can be used for addition of a fluorescent dye, purification of a polypeptide, and the like. The thus obtained VL-containing polypeptide and VH-containing polypeptide can be caused to form a complex in an appropriate solvent during, before, or after labeling with a fluorescent dye. Specifically the polypeptides are bound via a disulfide bond or using a cross-linking agent to form a complex, for example. For example, genes encoding the above VL-containing polypeptide and VH-containing polypeptide are co-expressed in an *Escherichia coli (E. coli)* cell-free synthesis system, followed by 16 hours of incubation at 4°C to form a disulfide bond. Thus, a complex can be formed. Moreover, molecular chaperon such as protein disulfide isomerase or proline cis/trans isomerase is added to an *E. coli* cell-free synthesis (reaction) system, and thus disulfide bonding can be accelerated. Moreover, the above cross-linking agent may be a compound that can cause the cross-linking and binding of polypeptides. Examples thereof include aldehydes (e.g., glutaraldehyde), carbodiimides, and imidoesters. A commercially available cross-linking agent can be adequately obtained and used according to a conventional method. Furthermore, the complex of the present invention can also be prepared by cleaving an antibody with an enzyme or the like. For example, an antibody is treated with papain or pepsin, and thus a Fab fragment or a F(ab')2 fragment can also be prepared.

In the present invention, a method for labeling a VL-containing polypeptide or a VH-containing polypeptide with a fluorescent dye is not particularly limited. A method for directly labeling using functional groups on both ends or a side chain of the polypeptide or indirectly labeling with a cross-linking agent or the like, a technique for site-specifically labeling while synthesizing polypeptides using a cell-free translation system, or the like can be used herein. As a method for labeling with the use of a cell-free translation system, an amber suppression method (Ellman J et al. (1991) Methods Enzymol.202: 301-36), a four-base codon method (Hohsaka T., et al., J. Am. Chem. Soc., 118, 9778-9779, 1996), a C-terminal labeling method (JP Patent Publication (Kokai) No. 2000-139468 A), an N-terminal labeling method (U.S. Patent No. 5,643,722, Olejnik et al. (2005) Methods 36: 252-260), or the like is known. The amber suppression method involves preparing DNA or mRNA by substituting a codon encoding an amino acid at a labeling target site with an amber codon that is one of termination codons, and then synthesizing a protein from the DNA or the mRNA using a cell-free translation system. At this time, suppressor tRNA to which a labeled non-natural amino acid has been bound is added to a reaction solution for protein synthesis, and thus a protein, in which such a labeled amino acid has been introduced into a site subjected to substitution with an amber codon, can be synthesized. The four-base codon method involves extending a codon mainly to a four-base codon, CGGG, preparing DNA or mRNA by substituting a codon encoding an amino acid with CGGG, and then synthesizing a protein from the DNA or the mRNA using a cell-free translation system. At this time, tRNA_{CGGG}, to which a labeled non-natural amino acid has been bound, is added to the reaction solution for protein synthesis, so that a protein, in which such a labeled amino acid has been introduced into a site subjected to substitution with the four-base codon can be synthesized. For the different-color double-label of the present invention, co-expression is performed by a combination of the amber suppression method and the four-base codon method using a cell-free translation system, a VH-containing polypeptide and a VL-containing polypeptide are labeled with different fluorescent dyes, and then a complex can be formed. According to the C-terminal labeling method, a cell-free translation system prepared by adding labeled puromycin at an optimum concentration, a protein is translated from DNA or mRNA, and the protein, in which a label has been introduced in a C-terminus specific manner, can be synthesized.

Moreover, a technique that involves site-specifically introducing a fluorescent dye by a gene-recombination technique using *Escherichia coli* or an animal cell as a host can also be used herein. Azidotyrosine is introduced site-specifically to a polypeptide using *Escherichia coli* as a host, into which aminoacyl tRNA synthase that recognizes azidotyrosine and suppressor azidotyrosyl-tRNA have been introduced. Then a fluorescent dye can be bound to the thus introduced azide group. Also, azide Z lysine is introduced site-specifically to a polypeptide using animal cells as host cells, into which archaebacteria-derived pyrrolidyl tRNA synthase and suppressor pyrrolidyl-tRNA have been introduced, and thus a fluorescent dye can be bound to the thus introduced azide group.

In the present invention, a fluorescent dye to be used for fluorescent labeling is not particularly limited, as long as it is a fluorescent dye that is quenched in the absence of an antigen under a condition where the fluoro-labeled complex of the present invention is formed, when a VH-containing polypeptide and/or a VL-containing polypeptide is labeled, and it emits fluorescence when such a complex and an antigen are bound to cancel the quenching functions. Also, when the same or different types of fluorescent dye are added to a VH-containing polypeptide and a VL-containing polypeptide, a combination is preferably selected so that, in addition to the above quenching, quenching between dyes and quenching resulting from the FRET effect effectively take place in the absence of an antigen. Examples of a fluorescent dye to be used for fluorescent labeling include fluorescent dyes having rhodamine, coumarin, Cy, EvoBlue, oxazine, carbopyronin, naphthalene, biphenyl, anthracene, phenenthrene, pyrene, carbazole, or the like as a backbone, or derivatives of such fluorescent dyes. Specific examples thereof include CR110: carboxyrhodamine 110: Rhodamine Green (trade name), TAMRA: carboxytetramethylrhodamine: TMR, carboxyrhodamine 6G: CR6G, ATTO 655 (trade name), BODIPY FL (trade name): 4,4-difluoro-5,7-dimethyl-4-bora-3a,4a-diaza-s-indancene-3-propionic acid, BODIPY 493/503 (trade name): 4,4-difluoro-1,3,5,7-tetramethyl-4-bora-3a,4a-diaza-s-indancene-8-propionic acid, BODIPY R6G (trade name): 4,4-difluoro-5-(4-phenyl-1,3-butadienyl) -4-bora-3a,4a-diaza-s-indancene-3-propionic acid, BODIPY 558/568 (trade name): 4,4-difluoro-5-(2-thienyl)-4-bora-3a,4a-diaza-s-indancene-3-propionic acid, BODIPY 564/570 (trade name): 4,4-difluoro-5-styryl-4-bora-3a,4a-diaza-s-indancene-3-propionic acid, BODIPY 576/589 (trade name): 4,4-difluoro-5-(2-pyrrolyl)-4-bora-3a,4a-diaza-s-indancene-3-propionic acid, BODIPY 581/591 (trade name): 4,4-difluoro-5-(4-phenyl-1, 3-butadienyl)-4-bora-3a,4a-diaza-s-indancene-3-propionic acid, Cy3 (trade name), Cy3B (trade name), Cy3.5 (trade name), Cy5 (trade name), Cy5.5 (trade name), EvoBlue10 (trade name), EvoBlue30 (trade name), MR121, ATTO 390 (trade name), ATTO 425 (trade name), ATTO 465 (trade name), ATTO 488 (trade name), ATTO 495 (trade name), ATTO 520 (trade name), ATTO 532 (trade name), ATTO Rho6G (trade name), ATTO 550 (trade name), ATTO 565 (trade name), ATTO Rho3B (trade name), ATTO Rho11 (trade name), ATTO Rho12 (trade name), ATTO Thio12 (trade name), ATTO 610 (trade name), ATTO 611X (trade name), ATTO 620 (trade name), ATTO Rho14 (trade name), ATTO 633 (trade name), ATTO 647 (trade name), ATTO 647N (trade name), ATTO 655 (trade name), ATTO Oxa12 (trade name), ATTO 700 (trade name), ATTO 725 (trade name), ATTO 740 (trade name), Alexa Fluor 350 (trade name), Alexa Fluor 405 (trade name), Alexa Fluor 430 (trade name), Alexa Fluor 488 (trade name), Alexa Fluor 532 (trade name), Alexa Fluor 546 (trade name), Alexa Fluor 555 (trade name), Alexa Fluor 568 (trade name), Alexa Fluor 594 (trade name), Alexa Fluor 633 (trade name), Alexa Fluor 647 (trade name), Alexa Fluor 680 (trade name), Alexa Fluor 700 (trade name), Alexa Fluor 750 (trade name), Alexa Fluor 790 (trade name), Rhodamine Red-X (trade name), Texas Red-X (trade name), 5 (6)-TAMRA-X (trade name), 5TAMRA (trade name), and SFX (trade name). In particular, particularly preferable examples thereof include rhodamine-based fluorescent dyes, such as CR110 and TAMRA, and an oxazine-based fluorescent dye such as ATTO 655.

A quencher to be used in the present invention is not particularly limited, as long as it can quench the fluorescence of a fluorescent dye that is used for labeling one of components (a VL-containing polypeptide and a VH-containing polypeptide) of the fluoro-labeled complex of the present invention in the absence of an antigen, when added to the other polypeptide, and its quenching function is canceled and fluorescence is emitted when the complex binds to an antigen. Examples of such a quencher include quenching dyes containing NBD: 7-nitrobenzofurazan, DABCYL, BHQ, ATTO, QXL, QSY, Cy, Lowa Black, IRDYE, and the like as backbones and derivatives thereof. Specific examples thereof include NBD, DABCYL, BHQ-1 (trade name), BHQ-2 (trade name), BHQ-3 (trade name), ATTO 540Q (trade name), ATTO 580Q (trade name), ATTO 612Q (trade name), QXL490 (trade name), QXL520 (trade name), QXL570 (trade name), QXL610 (trade name), QXL670 (trade name), QXL680 (trade name), QSY-35 (trade name), QSY-7 (trade name), QSY-9 (trade name), QSY-21 (trade name), Cy5Q (trade name), Cy7Q (trade name), Lowa Black FQ (trade name), Lowa Black RQ (trade name), and IRDYE QC-1 (trade name). Of these examples, NBD is preferred. Also, any combination of a fluorescent dye and a quencher in the complex of the present invention can be adequately selected, as long as the quencher effectively quenches the fluorescent dye in the absence of an antigen, but the emission of the fluorescent dye is not inhibited in the presence of an antigen. An example thereof is a combination of a fluorescent dye TAMRA and NBD.

In the case of the fluoro-labeled complex of the present invention; that is, a complex comprising a VL-containing polypeptide and a VH-containing polypeptide, either or both the VL-containing polypeptide and the VH-containing polypeptide are labeled with a fluorescent dye(s), the quenching of the fluorescent dye takes place in the absence of an antigen because of interaction between the above fluorescent dye and tryptophan residues conserved in the antibody variable region. In addition to this, in the case of the fluoro-labeled complex of the present invention, wherein the polypeptides are labeled with fluorescent dyes of the same color, the quenching effect between fluorescent dyes can be obtained. Moreover, in the case of the fluoro-labeled complex of the present invention, wherein the above polypeptides are labeled with fluorescent dyes of different colors, the quenching effect due to the fluorescence resonance energy transfer (FRET) effect can be obtained in addition to the above quenching due to tryptophan residues and quenching between the fluorescent dyes. Furthermore, in the case of the fluoro-labeled complex of the present invention, wherein the above polypeptides are labeled with a fluorescent dye and a quencher for quenching the fluorescent dye, respectively, the dynamic range can be increased by the quenching effect between the fluorescent dye and the quencher.

The method for measuring and/or detecting the concentration of an antigen of the present invention may be a method for measuring and/or detecting the concentration of an antigen which is characterized by comprising the following steps (a) to (c) in sequence:
(a) bringing a complex into contact with an antigen in a specimen for measurement, wherein the complex comprises a polypeptide containing an antibody light chain variable region and a polypeptide containing an antibody heavy chain variable region, in which either or both the polypeptide containing the antibody light chain variable region and the polypeptide containing the antibody heavy chain variable region are labeled with a fluorescent dye;
(b) detecting the fluorescence of the fluorescent dye, or measuring the fluorescence intensity of the fluorescent dye;
(c) calculating the amount of the antigen contained in a sample or visualizing the antigen using a positive correlation between the concentration of the antigen and the fluorescence intensity of the fluorescent dye as an indicator.
   Here, the above complex may be the fluoro-labeled complex of the present invention. In particular, examples thereof include the fluoro-labeled Fab complex of the present invention, more preferably the single-label Fab complex of the present invention, the same-color double-label Fab complex of the present invention, and the different-color double-label Fab complex of the present invention. In addition, the method for measuring and/or detecting the concentration of an antigen of the present invention can be performed using the fluoro-labeled complex of the present invention and the kit for measuring and/or detecting the concentration of an antigen of the present invention.

When the kit for measuring and/or detecting the concentration of an antigen of the present invention is used and the method for measuring and/or detecting the concentration of an antigen of the present invention is performed, the fluoro-labeled complex of the present invention is preferably brought into contact with an antigen in a liquid phase. Accordingly, a sample; that is a specimen to be measured is preferably prepared adequately as a liquid specimen or a specimen containing liquid, or a specimen to be measured, which is immersed in liquid and then subjected to the above step (a) or the method for measuring and/or detecting the concentration of an antigen of the present invention. The use of the kit for measuring and/or detecting the concentration of an antigen of the present invention, the origin of a sample to be subjected to the method for measuring and/or detecting the concentration of an antigen of the present invention, and the like are not particularly limited. Pretreatment and the like are adequately performed and thus the above specimen for measurement can be prepared. A liquid sample can be directly subjected to measurement as a specimen for measurement, or can be diluted with buffer, physiological saline, or the like, concentrated, or adequately adjusted to have a pH, a salt concentration, or the like to prepare a specimen for measurement, as long as an antigen is not deteriorated or the measurement and/or the detection of antigen concentration is not inhibited. Examples of such a liquid sample include body fluids that can contain a target antigen to be measured, such as serum, blood plasma, saliva, spinal fluids, and urine, culture supernatants, cell extracts, microbial extracts, and industrial wastewater.

A non-liquid sample such as a solid sample can be directly used as a specimen for measurement. Alternatively, such a solid sample may be adequately treated (e.g., divided, shredded, crushed, ground, prepared into tissue sections, or subjected to removal or extraction of only a specific component of the sample), as long as the antigen is not deteriorated or the measurement and/or detection of the concentration of the antigen is not inhibited, and then dissolved, suspended, or immersed in a liquid such as a buffer or physiological saline, so that the fluoro-labeled complex of the present invention can come into contact with the antigen, and then the resultant can be used as a specimen for measurement. In the above tissue section preparation, immobilization treatment can be performed using paraformaldehyde, glutaraldehyde or the like without deteriorating the antigen. Moreover, blocking treatment can also be performed using BSA (bovine serum albumin), skim milk or the like. Examples of such a solid sample include a nitrocellulose membrane or a PVDF membrane to which ingredients such as tissue, cells, proteins, and sugars (collected *in vivo)* have been blotted, foods and soil. Moreover, a specimen for measurement may adequately contain an antiseptic, a fungicide, a pH adjuster, a surfactant, an anticoagulant agent, a chelating agent, or the like, as long as it does not deteriorate the antigen and not inhibit the measurement and/or the detection of the concentration of the antigen.

In the present invention, furthermore, body fluids such as blood and spinal fluid, tissue, and the like *in vivo* can also be used as specimens for measurement. Specifically, the fluoro-labeled complex of the present invention is administered to a non-human animal such as an experimental animal, so that the fluoro-labeled complex of the present invention can be brought into contact with an antigen *in vivo.* Such a non-human animal may be any animal other than humans. Examples thereof include vertebrates and particularly, non-human animals such as mammals, fishes, birds, reptiles, and amphibians. In particular, mammals are preferred, and mice, rats, hamsters, monkeys, pigs, and the like are more preferred. Also, the above administration method is not particularly limited, and an administration method can be adequately selected from parenteral local administration methods including intramuscular injection, intraperitoneal injection, intravenous injection, subcutaneous injection, embedding, and coating, and oral administration methods. Moreover, another drug and the like may also be administered before, simultaneously with, or after the administration of the fluoro-labeled complex of the present invention. Through administration of the fluoro-labeled complex of the present invention to a non-human animal, the position or the transfer of an antigen *in vivo,* the amount of or changes in the amount of an antigen *in vivo* can also be observed. For such observation, samples such as body fluids and tissues are collected over time, specimens for measurement are prepared, and thus fluorescence intensity can be measured, localization of fluorescence can be observed, or *in vivo* fluorescence intensity and changes in *in vivo* fluorescence intensity, and localization and the transfer of fluorescence can be detected and observed in real time.

Reaction conditions for bringing the fluoro-labeled complex of the present invention into contact with an antigen in a specimen for measurement are not particularly limited, as long as they can be generally employed for an antigen-antibody reaction after the addition of the fluoro-labeled complex of the present invention to a specimen for measurement, followed by incubation thereof. The temperature conditions range from 1°C to 30°C, and preferably range from 18°C to 25°C, for example. The reaction time ranges from a second to 180 minutes, and preferably ranges from 1 to 90 minutes, for example. Furthermore, when a reaction is performed *in vivo* in a non-human animal, incubation is performed after administration for 5 to 180 minutes and preferably for 60 to 120 minutes, for example. If necessary, treatment such as, excision of tissue, blood, cells or the like, or exposition of an observation target site can be adequately performed. In a specimen after incubation, the quenching of the fluoro-labeled complex of the present invention that has recognized the antigen is canceled, fluorescence is emitted by irradiation with excitation light. However, the fluoro-labeled complex of the present invention that has not yet recognized the antigen remains being quenched, and no fluorescence is emitted even via irradiation with excitation light. Accordingly, a specimen for measurement, to which the above fluoro-labeled complex has been added can be directly subjected to the measurement and/or the detection of the concentration of an antigen without being subjected to a step such as a washing step. This is a significant characteristic of the kit for measuring and/or detecting the concentration of an antigen of the present invention and the method for measuring and/or detecting the concentration of an antigen of the present invention.

A method for detecting fluorescence in a specimen for measurement, which is employed in the present invention, is not particularly limited, as long as fluorescence emitted from a fluorescent dye can be detected, and a specimen for measurement after the above reaction is irradiated with excitation light and then the fluorescence intensity of the fluorescent dye can be measured and/or detected. Excitation light to be used for irradiation and the wavelength of fluorescence to be measured and/or detected can be adequately selected depending on the type of a fluorescent dye to be used herein. For example, when CR110 is used as a fluorescent dye, a combination of an excitation light wavelength of 480 nm and a fluorescence wavelength of 530 nm can be employed. When TAMRA is used, a combination of an excitation light wavelength of 530 nm and a fluorescence wavelength of 580 nm can be employed. When ATTO 655 is used, a combination of an excitation light wavelength of 630 nm and a fluorescence wavelength of 680 nm can be employed. Moreover, when different two types of fluorescent dye are used, a combination of an excitation light wavelength and a fluorescence wavelength, which enables the measurement of the concentration of an antigen and/or the detection of an antigen can be adequately selected and used. For example, a specimen for measurement caused to react with the fluoro-labeled complex of the present invention is irradiated with light with an excitation wavelength suitable for one of fluorescent dyes contained in the above fluoro-labeled complex, so as to obtain a fluorescence emission spectrum. This procedure is performed for both two types of fluorescent dye, so that a combination of an excitation light wavelength and a fluorescence wavelength optimum for the measurement and/or detection of the concentration of an antigen can be specified. An example of a combination of an excitation light wavelength and a fluorescence wavelength is a combination of an excitation light wavelength and a fluorescence wavelength, which is suitable for any one of fluorescent dyes. A more preferable example thereof is a combination of an excitation light wavelength and a fluorescence wavelength, which is suitable for a fluorescent dye with an excitation light wavelength and a fluorescence wavelength shorter than the other. In addition fluorescence may be detected as a fluorescence emission spectrum or fluorescence intensity at a specific wavelength. For example, when a combination of CR110 and TAMRA is used, fluorescence with a wavelength of 530 nm can be detected with an excitation light wavelength of 480 nm, and fluorescence can also be detected as a fluorescence emission spectrum with a wavelength ranging from 515 nm to 650 nm. With the use of a combination of an excitation light wavelength and a fluorescence wavelength, which is suitable for one of different two types of fluorescent dye, a reduction in the background resulting from the FRET (Fluorescence resonance energy transfer) effect in the absence of an antigen can be efficiently detected and measured with higher sensitivity.

A light source and a measuring device to be used for fluorescence detection in the present invention can be adequately selected. A light source may be any light source by which radiation with an excitation light wavelength is possible, and examples thereof include a mercury lamp, a xenon lamp, LED, and a laser beam. Excitation light with a specific wavelength can be obtained using an appropriate filter. A device to be generally used for fluorescence observation can be used as a fluorescence measuring device. A microscope and the like provided with an excitation light source, an irradiation system thereof, and a fluorescence image acquisition system can be adequately used, for example. Examples thereof include MF20/FluoroPoint-Light (Olympus Corporation) and FMBIO-III (Hitachi Software Engineering Co., Ltd.). Fluorescence intensity and the concentration of an antigen are in a positive correlation. Hence, fluorescence intensity is measured when a substance to be tested containing an antigen with a known concentration is used, a standard curve showing the relationship between the concentration of the antigen and the fluorescence intensity is created, and then the concentration of an antigen (with an unknown concentration) can be calculated from the standard curve. Regarding such calculation of the concentration of an antigen, the amount of an antigen can be automatically calculated using the conversion equation or the like determined based on the standard curve created in advance. In addition, the detection of fluorescence may be the detection of a fluorescence emission spectrum or the detection of fluorescence intensity at a specific wavelength.

Furthermore, when the fluoro-labeled complex of the present invention is administered to a non-human animal, a region to be detected of the non-human animal is irradiated with excitation light, and thus the fluorescence of a fluorescent dye can be measured and/or detected two-dimensionally or three-dimensionally, in addition to the collection of a body fluid, tissue, or the like thereof. In this case, a fluorescence microscope, a fluorescent image analyzer, an endoscope provided with a light source, and the like, can be used, for example. Moreover, when detection is performed, images showing the body, tissue, or cell structures of a non-human animal are also preferably obtained using an endoscope, X-ray, CT, MRI, ultrasonic wave, microscope, or the like. Measured and/or detected fluorescence intensities and the amounts of an antigen are in a positive correlation. Hence, based on the thus detected two-dimensional or 3-dimensional fluorescence images, the localization (position) and/or the amount of the antigen can be found, and the results can also be compared with the images showing the above structures, at this time. When fluorescence is detected, a specimen for measurement or the like containing no fluoro-labeled complex of the present invention or no analyte is preferably prepared as a negative control, and also preferably subjected to measurement and/or detection. Moreover, the amount of an antigen can also be measured using a fluorescence intensity ratio found by dividing a fluorescence level measured for a specimen for measurement by a fluorescence level measured for the negative control, for example. Alternatively, fluorescence intensities and the amounts of an antigen are in a positive correlation in the present invention, and thus when fluorescence intensity exceeding an adequately determined threshold is obtained, the presence of the antigen in a specimen for measurement can also be determined.

As described above, according to the present invention, all antigens that can be measured by immunoassays such as ELISA, immunodiffusion, latex agglutination, immunochromatography, a surface plasmon resonance method, and the like can be detected. For example, competitive ELISA is generally employed for performing an immunoassay of a low-molecular-weight substance. The method for detection and measurement of a low-molecular-weight substance according to the present invention is better than competitive ELISA in terms of convenient techniques, detection/measurement sensitivity, SN ratio, and the like, and is capable of exhibiting its best capacity. Examples of such low-molecular-weight substances that can be measured and/or detected by the method of the invention include: stimulant drugs and narcotics such as amphetamine, methamphetamine, morphine, heroin, and codeine; mycotoxins such as aflatoxin, sterigmatocystin, neosolaniol, nivalenol, fumonisin, ochratoxin, and endophyte-producing toxin; sex hormones such as testosterone and estradiol; additives that are illegally used for feedstuffs, such as clenbuterol and ractopamine; hazardous substances such as PCB, gossypol, histamine, benzpyrene, melamine, acrylamide, and dioxin; residual agricultural chemicals, such as acetamiprid, imidacloprid, chlorfenapyr, malathion, carbaryl, clothianidin, triflumizol, chlorothalonil, spinosad, methomyl (lannate), methamidophos, and clorpyrifos; and environmental hormones such as bisphenol A.

Furthermore, according to the present invention, measurement results can be instantly obtained and the detection method thereof is so simple that detection instruments can be downsized resulting in its lower price. These advantages can be exhibited not only for detection and/or measurement of low-molecular-weight substances, but also for on-site analyses by which measurement is performed on site. Furthermore, measurement is so easy according to the present invention, and thus not only a specialist but also a non-specialist can perform measurement. The method of the present invention can exhibit its ability thoroughly in the fields of: clinical diagnosis, specifically for detection and/or measurement of causative viruses and bacteria of influenza, communicable diseases, infectious diseases, and the like, blood drug levels, and POCT; simple medical examinations at workplaces, schools, day-care centers and home; security and safety, such as antiterrorism measures, specifically for detection and/or measurement of anthrax bacillus, botulinus toxin, sarin, and VX gas; environment, specifically for detection and/or measurement of environmental pollutants and house dust that should be detected and/or measured on site; and research and development requiring immunoassay, for example.

Hereafter, the present invention is described in greater detail with reference to the examples, although the technical scope of the present invention is not limited to the examples.

### Example 1

### 1. Establishment of homogenous fluorescence immunoassay using the fluoro-labeled complex of the present invention

### (Construction of expression vector)

### 1) Single-label Fab complex

A gene prepared by adding the DNA sequence of a ProX (trade name) tag (the nucleotide sequence corresponding to the 9^{th} amino acid is TTT, and MSKQIEVNFSNET; SEQ ID NO: 1 after translation) to the N-terminus and the DNA sequences of a linker (SEQ ID NO: 14) and a FLAG tag to the C-terminus of a DNA sequence encoding a polypeptide containing an anti-human osteocalcin (human Bone Gla Protein; BGP) antibody light chain variable region (VL; SEQ ID NO: 5) and an antibody light chain constant domain (Cκ; SEQ ID NO: 4) was incorporated into a pIVEX2.3d vector (Roche Diagnostics). A gene prepared by adding the DNA sequence of a ProX tag (the nucleotide sequence corresponding to the 9^{th} amino acid is TAG and MSKQIEVNXSNET (X denotes fluoro-labeled amino acid) after translation; SEQ ID NO: 2) containing an amber codon to the N terminus and the DNA sequences of a linker (SEQ ID NO: 14) and a His tag to the C-terminus of a DNA sequence encoding a polypeptide containing an anti-BGP antibody heavy chain variable region (VH; SEQ ID NO: 3) and an antibody heavy chain constant domain (CH₁; SEQ ID NO: 6) was incorporated into a pIVEX2.3d vector (Roche Diagnostics) (Fig. 5). The thus constructed expression vectors were designed so that the ProX tag (after translation, VH was labeled and VL was not labeled) was added to the N-terminus, and the His tag or the FLAG tag was added to the C-terminus of the inserted VL or VH.

A gene prepared by adding the DNA sequences of a ProX tag (SEQ ID NO: 1) and a GGGS5 spacer (GGGSGGGSGGGSGGGSGGGS; SEQ ID NO: 8) to the N-terminus and the DNA sequences of a linker (SEQ ID NO: 14) and a FLAG tag to the C-terminus of a DNA sequence encoding a polypeptide containing an anti-bisphenol A antibody light chain variable region (VL; SEQ ID NO: 7) and an antibody light chain constant domain (Cκ; SEQ ID NO: 4) was incorporated into a pIVEX2.3d vector (Roche Diagnostics). A gene prepared by adding the DNA sequences of a ProX tag containing an amber codon (SEQ ID NO: 2) and a GGGS5 spacer (SEQ ID NO: 8) to the N-terminus and the DNA sequences of a linker (SEQ ID NO: 14) and a His tag to the C-terminus of a DNA sequence encoding a polypeptide containing an anti-bisphenol A antibody heavy chain variable region (VH; SEQ ID NO: 9) and an antibody heavy chain constant domain (CH₁; SEQ ID NO: 6) was incorporated into a pIVEX2.3d vector (Roche Diagnostics) (Fig. 5). The thus constructed expression vectors were designed so that the ProX tag (after translation, VH was labeled and VL was not labeled) was added to the N-terminus and the His tag or the FLAG tag was added to the C-terminus of the inserted VL or VH.

A gene prepared by adding the DNA sequences of a ProX tag (SEQ ID NO: 1) and a GGGS2 spacer (GGGSGGGS; SEQ ID NO: 11) to the N-terminus and the DNA sequences of a linker (SEQ ID NO: 14) and a FLAG tag to the C-terminus of a DNA sequence encoding a polypeptide containing an anti-serum albumin (SA) antibody light chain variable region (VL; SEQ ID NO: 10) and an antibody light chain constant domain (Cκ; SEQ ID NO: 4) was incorporated into a pIVEX2.3d vector (Roche Diagnostics). Furthermore, a gene prepared by adding the DNA sequences of a ProX tag containing an amber codon (SEQ ID NO: 2) and a GGGS5 spacer (SEQ ID NO: 8) to the N-terminus and the DNA sequences of a linker (SEQ ID NO: 14) and a His tag to the C-terminus of a DNA sequence encoding a polypeptide containing an anti-serum albumin (SA) antibody heavy chain variable region (VH; SEQ ID NO: 12) and an antibody heavy chain constant domain (CH₁; SEQ ID NO: 6) was incorporated into a pIVEX2.3d vector (Roche Diagnostics) (Fig. 5). The thus constructed expression vectors were designed so that the ProX tag (after translation, VH was labeled, but VL was not labeled) was added to the N-terminus and the His tag or the FLAG tag was added to the C-terminus of the inserted VL or VH.

### 2) Same-color double-label Fab complex

A gene prepared by adding the DNA sequence of a ProX tag containing an amber codon (after translation, SEQ ID NO: 2) to the N-terminus and the DNA sequences of a linker (SEQ ID NO: 14) and a FLAG tag to the C-terminus of a DNA sequence encoding a polypeptide containing an anti-BGP antibody light chain variable region (VL; SEQ ID NO: 5) and an antibody light chain constant domain (Cκ; SEQ ID NO: 4) was incorporated into a pIVEX2.3d vector. A gene prepared by adding the DNA sequence of a ProX tag (after translation, SEQ ID NO: 2) containing an amber codon to the N-terminus and the DNA sequences of a linker (SEQ ID NO: 14) and a His tag to the C-terminus of a DNA sequence encoding a polypeptide containing an anti-BGP antibody heavy chain variable region (VH; SEQ ID NO: 3) and an antibody heavy chain constant domain (CH₁; SEQ ID NO: 6) was incorporated into a pIVEX2.3d vector. The thus constructed expression vectors were designed so that the ProX tag (amber) was added to the N-terminus and the His tag or the FLAG tag was added to the C-terminus of the inserted VL or VH.

A gene prepared by adding the DNA sequences of a ProX tag containing an amber codon (after translation, SEQ ID NO: 2) and a GGGS2 spacer (SEQ ID NO: 11) to the N-terminus and the DNA sequences of a linker (SEQ ID NO: 14) and a FLAG tag to the C-terminus of a DNA sequence encoding a polypeptide containing an anti-serum albumin (SA) antibody light chain variable region (VL; SEQ ID NO: 10) and an antibody light chain constant domain (Cκ; SEQ ID NO: 4) was incorporated into a pIVEX2.3d vector (Roche Diagnostics). Moreover, a gene prepared by adding the DNA sequences of a ProX tag containing an amber codon (after translation, SEQ ID NO: 2) and a GGGS2 spacer (SEQ ID NO: 11) to the N-terminus and the DNA sequences of a linker (SEQ ID NO: 14) and a His tag to the C-terminus of a DNA sequence encoding a polypeptide containing an anti-SA antibody heavy chain variable region (VH; SEQ ID NO: 12) and an antibody heavy chain constant domain (CH₁; SEQ ID NO: 6) was incorporated into a pIVEX2.3d vector. The thus constructed expression vector was designed so that the ProX tag (amber) was added to the N-terminus and the His tag or the FLAG tag was added to the C-terminus of the inserted VL or VH.

### 3) Different-color double-label Fab complex

A gene prepared by adding the DNA sequence of a ProX tag containing a CGGG four-base codon (the nucleotide sequence corresponding to the 9^{th} amino acid is CGGG; and SEQ ID NO: 2 after translation) to the N-terminus and the DNA sequences of a linker (SEQ ID NO: 14) and a FLAG tag to the C-terminus of a DNA sequence encoding a polypeptide containing an anti-BGP antibody light chain variable region (VL; SEQ ID NO: 5) and an antibody light chain constant domain (Cκ; SEQ ID NO: 4) was incorporated into a pIVEX2.3d vector. Moreover, a gene prepared by adding the DNA sequence of a ProX tag containing an amber codon (after translation, SEQ ID NO: 2) to the N-terminus and the DNA sequences of a linker (SEQ ID NO: 14) and a His tag to the C-terminus of a DNA sequence encoding a polypeptide containing an anti-BGP antibody heavy chain variable region (VH; SEQ ID NO: 3) and an antibody heavy chain constant domain (CH₁; SEQ ID NO: 6) was incorporated into a pIVEX2.3d vector (Fig. 5). The thus constructed expression vectors were designed so that the ProX tag was added to the N-terminus and the His tag or the FLAG tag was added to the C-terminus of the inserted VL or VH.

A gene prepared by adding the DNA sequences of a ProX tag containing a CGGG four-base codon (after translation, SEQ ID NO: 2) and a GGGS2 spacer (SEQ ID NO: 11) to the N-terminus and the DNA sequences of a linker (SEQ ID NO: 14) and a FLAG tag to the C-terminus of a DNA sequence encoding a polypeptide containing an anti-serum albumin (SA) antibody light chain variable region (VL; SEQ ID NO: 10) and the antibody light chain constant domain (Cκ; SEQ ID NO: 4) was incorporated into a pIVEX2.3d vector (Roche Diagnostics). Furthermore, a gene prepared by adding the DNA sequences of a ProX tag containing an amber codon (after translation, SEQ ID NO: 2) and a GGGS2 spacer (SEQ ID NO: 11) to the N-terminus and the DNA sequences of a linker (SEQ ID NO: 14) and a His tag to the C-terminus of a DNA sequence encoding a polypeptide containing an anti-SA antibody heavy chain variable region (VH; SEQ ID NO: 12) and an antibody heavy chain constant domain (CH₁; SEQ ID NO: 6) was incorporated into a pIVEX2.3d vector. The thus constructed expression vectors were designed so that the ProX tag was added to the N-terminus and the His tag or the FLAG tag was added to the C-terminus of the inserted VL or VH.

### (Synthesis of fluoro-labeled Fab complex)

Fluoro-labeled amino acids were introduced into an antibody-variable-region-containing peptide and/or the N-terminal region of an antibody-variable-region-containing peptide based on a cell-free translation system using an RYTS (Trade name) E. *coli* cell-free synthesis kit (Remarkable Yield Translation System Kit (ProteinExpress))

### 1) Single-label or same-color double-label Fab complex

A reaction solution (60 µL) was prepared by the addition of 3 µL of Enzyme Mix, 0.6 µL of methionine, 30 µL of 2×Reaction Mix, 20 µL of an E. *coli* lysate, 2 µL of two types of plasmid DNA (200 ng each), 3 µL of a fluoro-labeled amino acyl-tRNA amber (480 pmol), and 1.4 µL of Nuclease Free Water. CloverDirect (trade name) tRNA Reagents for Site-Directed Protein Functionalization (ProteinExpress) was used as fluoro-labeled aminoacyl-tRNA (TAMRA-X-AF-tRNA amber, CR110-X-AF-tRNA amber, and ATTO 655-X-AF-tRNA amber) for preparation of fluoro-labeled proteins. The reaction solution was left to stand at 20°C for 2 hours for reaction, and thus protein synthesis was performed. Thereafter, complex formation was completed by further 16 hours of reaction at 4°C. After the completion of the reaction, 0.5 µL of the reaction solution was used to perform SDS-PAGE (15%), and then protein expression was observed with a fluorescent image analyzer (FMBIO-III; Hitachi Software Engineering Co., Ltd.). Furthermore, Western blotting was performed using an anti-His tag antibody or an anti-FLAG tag antibody, thereby confirming the synthesis of the target peptide containing the fluoro-labeled antibody variable region.

### 2) Different-color double-label Fab complex

A reaction solution (60 µL) was prepared by the addition of 3 µL of Enzyme Mix, 0.6 µL of methionine, 30 µL of 2 x Reaction Mix, 20 µL of *E*. *coli* lysate, 2 µL of two types of plasmid DNA (200 ng each), 1.5 µL of two types of fluoro-labeled aminoacyl-tRNA amber and CGGG (480 nmol each), 1.4 µL of Nuclease Free Water. CloverDirect (trade name) tRNA Reagents for Site-Directed Protein Functionalization (ProteinExpress) were used as fluoro-labeled aminoacyl-tRNA (TAMRA-X-AF-tRNA amber or CGGG, CR110-X-AF-tRNA amber or CGGG, and ATTO 655-X-AF-tRNA amber or CGGG) for preparation of fluoro-labeled proteins. A VH-region-containing polypeptide and a VL-region-containing polypeptide were labeled with a tRNA_{amber} and tRNA_{CGGG}, respectively. The reaction solution was left to stand at 20°C for 2 hours to perform a reaction. After protein synthesis, complex formation was competed by further 16 hours of reaction at 4°C. After the completion of the reaction, 0.5 µL of the reaction solution was used to perform SDS-PAGE (15%), and then protein expression was observed with a fluorescent image analyzer (FMBIO-III; Hitachi Software Engineering Co., Ltd.). Furthermore, Western blotting was performed using an anti-His tag antibody or an anti-FLAG tag antibody, thereby confirming the synthesis of the target peptide containing the fluoro-labeled antibody variable region.

### 3) Fab complex comprising a polypeptide labeled with a fluorescent dye and the other polypeptide labeled with a quencher for quenching the fluorescent dye

TAMRA was used as a fluorescent dye and NBD-X, SE (Anspec) was used as a quencher. NBD-X-AF-tRNA CGGG was synthesized using NBD-X, SE (Anspec) instead of TAMRA-X, SE of Abe et al.,'s method (Abe R, et al., J. Biosci. Bioeng. 110 (1): 32-38 (2010)). A reaction solution (60 µL) was prepared by the addition of 3 µL of Enzyme Mix, 0.6 µL of metionine, 30 µL of 2xReaction Mix, 20 µL of *E*. *coli* lysate, 2 µL of two types of plasmid DNA (200 ng each), 1.5 µL of TAMRA-X-AF-tRNA amber and NBD-X-AF-tRNA CGGG (480 nmol each), and 1.4 µL of Nuclease Free Water. A VH region-containing polypeptide and a VL region-containing polypeptide were labeled with tRNA_{amber} and tRNA_{CGGG}, respectively. The reaction solution was left to stand at 20°C for 2 hours for reaction. After protein synthesis, complex formation was completed by further 16 hours of reaction at 4°C. After the completion of the reaction, 0.5 µL of the reaction solution was used to perform SDS-PAGE (15%), and then protein expression was observed with a fluorescent image analyzer (FMBIO-III; Hitachi Software Engineering Co., Ltd.). Moreover, Western blotting was performed using an anti-His tag antibody or an anti-FLAG tag antibody, thereby confirming the synthesis of the target peptide containing the fluoro-labeled antibody variable region.

### (Purification of fluoro-labeled Fab complex)

The thus synthesized fluoro-labeled Fab complexes were each purified using anti-FLAG M2 affinity gel (Sigma-Aldrich Corporation) or His-Spin Trap Column (GE Healthcare). The reaction solution (60 µL) was applied to a column containing anti-FLAG M2 affinity gel. After 15 minutes of incubation at room temperature, the resultant was washed 3 times with a wash buffer (20 mM phosphate buffer (pH 7.4)/0.5M NaCl/0.1% polyoxyethylene (23) lauryl ether). Next, the resultant was eluted 3 times with 200 µL of elute buffer (20 mM phosphate buffer (pH7.4)/0.5 M NaCl/100 µg FLAG peptide/0.1% polyoxyethylene (23) lauryl ether). Next, the eluate was applied to a His-Spin Trap Column. After 15 minutes of incubation at room temperature, the resultant was washed 3 times with a wash buffer (20 mM phosphate buffer (pH 7.4)/0.5 M NaCl/60 mM imidazole/0.1% polyoxyethylene (23) lauryl ether). Next, the resultant was eluted 3 times with 200 µL of an Elute buffer (20 mM phosphate buffer (pH7.4)/0.5 M NaCl/0.5 M imidazole/0.1% polyoxyethylene (23) lauryl ether). Furthermore, the eluate was subjected to buffer exchange and concentration using Amicon Ultra-0.5 centrifugal filter 10 kDa (Millipore) and PBS (+0.05% Tween20). The concentration of each sample after purification was measured using a fluorescent image analyzer (FMBIO-III; Hitachi Software Engineering Co., Ltd.).

### (Preparation of Q-body)

Single-chain antibodies (scFv) were prepared by linking the VH and VL (VH was labeled, but VL was not labeled) of a TAMRA-labeled anti-BGP antibody, and the VH and VL of TAMRA-labeled anti-BGP and the VH and VL of TAMRA-labeled anti-bisphenol A antibodies using a linker (GGGGSGGGGSGGGGS) according to the method described in International Publication WO2011/061944.

### Example 2

### 2. Measurement by homogenous fluorescence immunoassay using the fluoro-labeled complex of the present invention

### (Fluorescence emission spectrum measurement using single-label Fab complex)

The TAMRA single-label anti-BGP antibody Fab complex, the TAMRA-labeled anti-BGP antibody scFv, or the TAMRA-labeled anti-BGP antibody VH + anti-BGP antibody VL prepared in Example 1, comprising a polypeptide containing an anti-BGP (human osteocalcin) antibody light chain variable region (VL; SEQ ID NO: 5) and an antibody light chain constant domain (Cκ; SEQ ID NO: 4), and a polypeptide containing a TAMRA fluorescence-labeled anti-BGP antibody heavy chain variable region (VH; SEQ ID NO: 3) and an antibody heavy chain constant domain (CH₁; SEQ ID NO: 6) was used to measure the concentration of BGP. The TAMRA single-label anti-BGP antibody Fab complex, or the TAMRA-labeled anti-BGP scFv (70 nM, 6.25 µL), or the TAMRA-labeled anti-BGP antibody VH + anti-BGP antibody VL (70 nM/mL, 6.25 µL) and antigenic BGP-C7 (SEQ ID NO: 13) (0, 1, 3, 10, 25, 100, or 1,000 nM) were prepared to a total of 50 µL using PBS containing 1% BSA (+0.05% Tween20). The solution was left to stand at 25°C for 70 minutes, and then subjected to fluorescence emission spectrum measurement using a spectrophotofluorometer (FluoroMax-4; HORIBA Jobin Yvon). A He-Ne laser was used at 543 nm, with the excitation wavelength set at 530 nm, and the fluorescence intensity at 580 nm was measured. The ratio of the fluorescence intensity at each antigen concentration to the fluorescence intensity when no antigen was present is shown in the upper left graph of Fig. 6. The ratio of the fluorescence intensity when the concentration of BGP-C7 was 1,000 nM is shown in the lower left table of Fig. 6. Similarly, the TAMRA single-label anti-bisphenol A Fab complex, or the TAMRA-labeled anti-bisphenol A antibody scFv, comprising a polypeptide containing a TAMRA fluorescence-labeled anti-bisphenol A antibody VL (SEQ ID NO: 7) and Cκ (SEQ ID NO: 4) and a polypeptide containing an anti-bisphenol A antibody VH (SEQ ID NO: 9) and CH₁ (SEQ ID NO: 6) was reacted with bisphenol A (0, 1, 3, 10, 30, 100, or 1,000 nM), and then fluorescence intensity was measured. The ratio of the fluorescence intensity at each antigen concentration to the fluorescence intensity when no antigen was present is shown in the upper right graph of Fig. 6. The ratio of the fluorescence intensity when the concentration of bisphenol A was 1,000 nM is shown in the lower right table of Fig. 6. It was confirmed that the single-label Fab complex of the present invention makes it possible to obtain a fluorescence intensity ratio higher than that obtained using conventional scFv-type Q-body or VH + VL-type Q-body, regardless of the type of fluorescent dye or antigen concentration, and to detect and quantify a low-molecular-weight compound and a protein with high sensitivity. The Fab complex of the present invention forms a complex wherein its components; that is, polypeptides, are located close to each other, compared with those in the scFv-type Q-body or the VH + VL-type Q-body, so that the quenching effect of tryptophan in an antibody variable region on a fluorescent dye takes place effectively.

### Example 3

### (Fluorescence emission spectrum measurement using same-color double-label Fab complex)

The same-color double-label Fab complex (70 nM, 6.25 µL) prepared in Example 1, comprising a polypeptide containing a light chain variable region (VL; SEQ ID NO: 5) of an anti-BGP antibody labeled with CR110, TAMRA, or ATTO 655 fluorescent dye and an antibody light chain constant domain (Cκ; SEQ ID NO: 4) and a polypeptide containing a heavy chain variable region (VH; SEQ ID NO: 3) of an anti-BGP antibody labeled with the same dye and an antibody heavy chain constant domain (CH₁; SEQ ID NO: 6) and antigenic BGP-C7 (0 to 10,000 nM) were prepared to a total of 50 µL with PBS containing 1% BSA (+0.05% Tween20). As a control, a single-label Fab complex sample wherein either a polypeptide containing anti-BGP antibody VL (SEQ ID NO: 5) and Cκ (SEQ ID NO: 4) or a polypeptide containing anti-BGP antibody VH (SEQ ID NO: 3) and CH₁ (SEQ ID NO: 6) had been fluoro-labeled was prepared. These solutions were left to stand at 25°C for 70 minutes, and then the fluorescence emission spectrum was measured using a spectrophotofluorometer (FluoroMax-4; HORIBA Jobin Yvon). When the same-color double-label Fab complex labeled with a CR110 fluorescent dye was used, the excitation wavelength (Ex) was set at 480 nm, and then the fluorescence intensity was measured at a fluorescence wavelength (Em) of 530 nm. When the same-color double-label Fab complex labeled with a TAMRA fluorescent dye was used, the excitation wavelength was set at 530 nm, and then the fluorescence intensity was measured at a fluorescence wavelength of 580 nm. When the same-color double-label Fab complex labeled with an ATTO 655 fluorescent dye was used, the excitation wavelength was set at 630 nm, and then the fluorescence intensity was measured at a fluorescence wavelength of 680 nm. The ratio of the fluorescence intensity at each antigen concentration to the fluorescence intensity when no antigen was present was designated as "fluorescence intensity ratio" and is shown in the graphs of Fig. 7. Fluorescence intensity ratios when the concentration of BGP-C7 was 1,000 nm or 10,000 nm are shown in the tables of Fig. 7.

In a similar manner, the same-color double-label Fab complex comprising a polypeptide containing an anti-serum albumin (SA) antibody light chain variable region (VL; SEQ ID NO: 10) and an antibody light chain constant domain (Cκ; SEQ ID NO: 4) and a polypeptide containing a heavy chain variable region (VH; SEQ ID NO: 12) of an anti-SA antibody labeled with the same dye and an antibody heavy chain constant domain (CH₁; SEQ ID NO: 6) was reacted with antigenic HSA (0 to 100 µM), and then fluorescence intensity was measured. Fluorescence intensity ratios are shown in the graphs of Fig. 8 and fluorescence intensity ratios when the concentration of HSA was 100 µM are shown in the tables of Fig. 8. Based on the above results, it was confirmed that the same-color double-label Fab complex of the present invention makes it possible to measure the amount of an antigen with a fluorescence intensity ratio higher than that of the single-label Fab complex, regardless of the type of fluorescent dye and the type of antigen used. The same-color double-label Fab complex can reduce the background so as to enhance the dynamic range because of the quenching effect (H-dimer) due to the interaction between fluorescent dyes, in addition to the quenching effect of tryptophan in an antibody variable region on a fluorescent dye(s).

### Example 4

### (Fluorescence emission spectrum measurement using different-color double-label Fab complex)

The different-color double-label Fab complex (70 nM, 6.25 µL) of an anti-BGP antibody prepared in Example 1, comprising a polypeptide containing a light chain variable region (VL; SEQ ID NO: 5) of an anti-BGP antibody labeled with CR110, TAMRA, or ATTO 655 fluorescent dye and an antibody light chain constant domain (Cκ; SEQ ID NO: 4) and a polypeptide containing a heavy chain variable region (VH; SEQ ID NO: 3) of an anti-BGP antibody labeled with a dye differing from the above dye and an antibody heavy chain constant domain (CH₁; SEQ ID NO: 6) and antigenic BGP-C7 (0 to 1,000 nM) were prepared to a total of 50 µL using PBS (+0.05% Tween20) containing 1% BSA. As a control, a same-color double-label Fab complex sample was prepared. Fluorescence intensity was assumed in a manner similar to Example 3. The ratio of the fluorescence intensity at each antigen concentration to the fluorescence intensity when no antigen was present was obtained. In addition, when CR110 and TAMRA were used, the excitation wavelength was set at 480 nm, and then the fluorescence intensity was measured at a fluorescence wavelength of 530 nm (the left graph of Fig. 9). When CR110, TAMRA, and ATTO 655 were used, the excitation wavelength was set at 530 nm, and then the fluorescence intensity was measured at a fluorescence wavelength of 580 nm (the center graph of Fig. 9). When TAMRA and ATTO 655 were used, the excitation wavelength was set at 630 nm, and then the fluorescence intensity was measured at a fluorescence wavelength of 680 nm (the right graph of Fig. 9). Fluorescence intensity ratios when the concentration of BGP-C7 was 1,000 nM are shown in each table of Fig. 9.

Similarly, the different-color double-label Fab complex of the anti-SA antibody prepared in Example 1 comprising a polypeptide containing a heavy chain variable region (VH; SEQ ID NO: 12) of an anti-SA antibody labeled with a CR110, TAMRA, or ATTO 655 fluorescent dye and an antibody heavy chain constant domain (CH₁; SEQ ID NO: 6), and a polypeptide containing a light chain variable region (VL; SEQ ID NO: 10) of an anti-SA antibody labeled with a dye differing from the above dye and an antibody light chain constant domain (Cκ; SEQ ID NO: 4) was reacted with antigenic HSA (0 to 100 µM), and then fluorescence intensity was measured. The ratio of the fluorescence intensity at each antigen concentration to the fluorescence intensity when no antigen was present was designated as the "fluorescence intensity ratio" and is shown in the graphs of Fig. 10. Fluorescence intensity ratios when the concentration of HSA was 100 µM are shown in the tables of Fig. 10.

Furthermore, the different-color double-label Fab complex (also denoted as CR110_TAMRA) of an anti-SA antibody, comprising a polypeptide containing a CR110-labeled anti-SA antibody heavy chain variable region (VH; SEQ ID NO: 12) and an antibody heavy chain constant domain (CH₁; SEQ ID NO: 6) and a polypeptide containing a TAMRA-labeled anti-SA antibody light chain variable region (VL; SEQ ID NO: 10) and an antibody light chain constant domain (Cκ; SEQ ID NO: 4) was reacted with antigenic HSA (1×10⁻⁴, 1×10⁻⁵, 1×10⁻⁶, 1×10⁻⁷M). The resultant was irradiated with excitation light having a wavelength of 480 nm (the upper left graph of Fig. 11) or a wavelength of 530 nm (the upper right graph of Fig. 11), and then the fluorescence emission spectrum was measured. Moreover, the CR110 single-label Fab complex (CR110_no) of an anti-SA antibody, comprising a polypeptide containing CR110-labeled anti-SA antibody VH (SEQ ID NO: 12) and CH₁ (SEQ ID NO: 6) and a polypeptide containing anti-SA antibody VL (SEQ ID NO: 10) and Cκ (SEQ ID NO: 4) was reacted with antigenic HSA (1×10⁻⁴, 1×10⁻⁵, 1×10⁻⁶, 1×10⁻⁷M). The resultant was irradiated with an excitation light having a wavelength of 480 nm, and then the fluorescence emission spectrum was measured (the lower left graph of Fig. 11). The TAMRA single-label Fab complex (no_TAMRA) of an SA antibody comprising a polypeptide containing anti-SA antibody VH (SEQ ID NO: 12) and CH₁ (SEQ ID NO: 6) and a polypeptide containing TAMRA-labeled anti-SA antibody VL (SEQ ID NO: 10) and Cκ (SEQ ID NO: 4) was reacted with antigenic HSA (1×10⁻⁴, 1×10⁻⁵, 1×10⁻⁶, 1×10⁻⁷M). The resultants were irradiated with excitation light with a wavelength of 530 nm and then the fluorescence emission spectrum was measured using a spectrophotofluorometer (FluoroMax-4) (the lower right graph of Fig. 11). The curves in each graph are of data obtained from samples with HSA concentrations of (from the top) 1×10⁻⁴ M, 1×10⁻⁵ M, 1×10⁻⁶M, 1×10⁻⁷M, and 0 M.

The above results revealed that in the case of the different-color double-label Fab complex CR110_TAMRA, fluorescence at a wavelength of about 530 nm in the absence of an antigen was suppressed by the FRET effect, when irradiated with excitation light with a wavelength of 480 nm, and thus a high fluorescence intensity ratio was obtained at a wavelength of about 530 nm. Moreover, when irradiated with excitation light with a wavelength of 530 nm, fluorescence intensity ratios obtained at a fluorescence wavelength of about 530 nm in the case of CR110_TAMRA was higher than those of no_TAMRA, because of the quenching effect due to the interaction between the fluorescent dyes. Therefore, the background can be reduced by the addition of the quenching effect due to the interaction between fluorescent dyes and the FRET effect, in addition to the quenching effect of tryptophan in an antibody variable region on a fluorescent dye(s), thereby enhancing the dynamic range. In this experimental system, CR110_TAMRA was capable of detecting an antigen, while exhibiting a maximum of 75-fold fluorescence enhancement, thereby demonstrating the usefulness of the present invention.

### Example 5

### (Fluorescence emission spectrum measurement using Fab complex comprising fluoro-labeled polypeptide and quencher-labeled polypeptide)

The Fab complex (70 nM, 6.25 µL) prepared in Example 1, consisting of a polypeptide containing a TAMRA-labeled anti-BGP antibody heavy chain variable region (VH; SEQ ID NO: 3) and an antibody heavy chain constant domain (CH₁; SEQ ID NO: 6), and a polypeptide containing a NBD-labeled anti-BGP antibody light chain variable region (VL; SEQ ID NO: 5) and an antibody light chain constant domain (Cκ; SEQ ID NO: 4), and antigenic BGP-C7 (0 to 10,000 nM) were prepared to a total of 50 µL with PBS (+0.05% Tween20) containing 1% BSA. The solution was left to stand at 25°C for 70 minutes, and then the fluorescence emission spectrum was measured using a spectrophotofluorometer (FluoroMax-4; HORIBA Jobin Yvon). The excitation wavelength was set at 530 nm, and then the fluorescence intensity was measured at a fluorescence wavelength of 580 nm. The ratio of the fluorescence intensity at each antigen concentration to the fluorescence intensity when no antigen was present was designated as "fluorescence intensity ratio" and shown in the graph of Fig. 12. The fluorescence intensity ratio when the concentration of BGP-C7 was 1,000 nM is shown in the table of Fig. 12.

The usefulness of the present invention was demonstrated by the results that the Fab complex TAMRA_NBD was capable of detecting BGP at a concentration of 1 nM, the same as that in the case of the single-label Fab complex (TAMRA_No) (Fig. 7), and detecting an antigen, while exhibiting a 27-fold fluorescence enhancement, which was higher than that of the double-label (TAMRA_TAMRA) (Fig. 7).

### Example 6

### (Measurement of thermostability)

A thermal shift assay was performed to measure the thermal stability of the anti-BGP_TAMRA single-label Fab complex and the anti-BGP_TAMRA-labeled scFv prepared in Example 1. The temperature was increased by 1°C per minute using a StepOne real-time PCR system (Applied Biosystems), and then fluorescence intensity was measured at each temperature. Thermal stability was measured based on the principle that a quenching state is canceled when each antibody undergoes thermal denaturation to result in the disintegration of the structure, so that fluorescence intensity increases. As shown in Fig. 13, the Tm value (at which thermal denaturation takes place) for the TAMRA-labeled scFv was 61°C, however, the Tm value for the TAMRA single-label Fab complex of the present invention was 73°C; an increase of 12°C. This thermal stability enables the long-term storage of reagents and can significantly contribute to industrial merits such as distribution temperature, storage temperature, and storage period.

### Example 7

### (Measurement of clenbuterol using fluoro-labeled Fab complex)

The different-color double-label Fab complex synthesized by the method shown in Example 1, comprising a polypeptide containing an anti-clenbuterol antibody CR110-labeled light chain variable region (VL; SEQ ID NO: 15) and an antibody light chain constant domain (Cκ; SEQ ID NO: 4) and a polypeptide containing a TAMRA-labeled anti-clenbuterol antibody heavy chain variable region (VH; SEQ ID NO: 16) and an antibody heavy chain constant domain (CH₁; SEQ ID NO: 6) was reacted with antigenic clenbuterol (0 to 16 µg/mL), and then fluorescence intensity was measured following the method of Example 4. Fluorescence intensity ratio when the concentration of clenbuterol was 16 µg/mL is shown in Table 1. As a result, 16 µg/mL clenbuterol could be measured.

**[Table 1]**

| Measurement of clenbuterol using fluoro-labeled Fab complex | | | | | |
|---|---|---|---|---|---|
| Fluoro-labeled Fab antibody | Antigen | Labeling fluorescent dye | | Measurement | Fluorescence enhancement^{*1} |
| | | VH-containing polypeptide | VL-containing polypeptide | Ex/Em | |
| Anti-Clenbuterol | Clenbuterol | TAMRA | CR110 | 480/530 | 1.5 |

| | | | | | |
|---|---|---|---|---|---|
| ^{*1} (Fluorescence intensity in the presence of 16 µg/mL Clenbuterol)/(Fluorescence intensity in the absence of antigen) | | | | | |

### Example 8

### (Measurement of ractopamine and cotinine using fluoro-labeled Fab complex)

A same-color double-label Fab complex comprising a polypeptide containing an anti-ractopamine antibody TAMRA-labeled light chain variable region (VL; SEQ ID NO: 17) and an antibody light chain constant domain (Cκ; SEQ ID NO: 4) and a polypeptide containing a TAMRA-labeled anti-ractopamine antibody heavy chain variable region (VH; SEQ ID NO: 18) and an antibody heavy chain constant domain (CH₁; SEQ ID NO: 6) was synthesized by the method described in Example 1. Furthermore, a same-color double-label Fab complex comprising a polypeptide containing an anti-cotinine antibody TAMRA-labeled light chain variable region (VL; SEQ ID NO: 19) and an antibody light chain constant domain (Cκ; SEQ ID NO: 4) and a polypeptide containing a TAMRA-labeled anti-cotinine antibody heavy chain variable region (VH; SEQ ID NO: 20) and an antibody heavy chain constant domain (CH₁; SEQ ID NO: 6) was synthesized. Subsequently, each complex was reacted with antigenic ractopamine (3.4 µg/mL) or cotinine (3.5 µg/mL), and then fluorescence intensity was measured following the method of Example 4. The results are shown in Table 2. As shown in Table 2, measurement was successfully performed with fluorescence intensity ratios (indicating fluorescence enhancement) of 2.3 and 1.9.

**[Table 2]**

| Measurement of ractopamine and cotinine using fluoro-labeled Fab complex | | | | | |
|---|---|---|---|---|---|
| Fluoro-labeled Fab antibody | Antigen | Labeling fluorescent dye | | Measurement | Fluorescence enhancement |
| | | VH-containing polypeptide | VL-containing polypeptide | Ex/Em | |
| Anti-Ractopamine | Ractopamine | TAMRA | TAMRA | 530/580 | 2.3^{*1} |
| Anti-cotinine | Cotinine | TAMRA | TAMRA | 530/580 | 1.9^{*2} |

| | | | | | |
|---|---|---|---|---|---|
| ^{*1} (Fluorescence intensity in the presence of 3.4 µg/mL Ractopamine)/(Fluorescence intensity in the absence of antigen) ^{*2} (Fluorescence intensity in the presence of 3.5 µg/mL Cotinine)/(Fluorescence intensity in the absence of antigen) | | | | | |

### Example 9

### (Measurement of influenza A virus hemagglutinin (HA) using fluoro-labeled Fab complex)

A different-color double-label Fab complex synthesized by the method described in Example 1, comprising a polypeptide containing an anti-HA (hemagglutinin of influenza A (H5N1, H1N1)) antibody CR110-labeled light chain variable region (VL; SEQ ID NO: 21) and an antibody light chain constant domain (Cκ; SEQ ID NO: 4) and a polypeptide containing a TAMRA-labeled anti-HA (hemagglutinin of influenza A (H5N1, H1N1)) antibody heavy chain variable region (VH; SEQ ID NO: 22) and an antibody heavy chain constant domain (CH₁; SEQ ID NO: 6) was reacted with each antigen (30 µg/mL), and then fluorescence intensity was measured following the method of Example 4. The results are shown in Table 3. As shown in Table 3, measurement was successfully performed with fluorescence intensity ratios (indicating fluorescence enhancement) for H5N1 HA and H1N1 HA antigens were 6.1 and 7.1, respectively.

**[Table 3]**

| Measurement of influenza A virus HA using fluoro-labeled Fab complex | | | | | |
|---|---|---|---|---|---|
| Fluoro-labeled Fab antibody | Antigen | Labeling fluorescent dye | | Measurement | Fluorescence enhancement^{*1} |
| | | VH-containing polypeptide | VL-containing polypeptide | Ex/Em | |
| Anti-HA | H5N1 HA | TAMRA | CR110 | 480/530 | 6.1 |
| | H1N1 HA | TAMRA | CR110 | 480/530 | 7.1 |

| | | | | | |
|---|---|---|---|---|---|
| ^{*1} (Fluorescence intensity in the presence of 30 µg/mL antigen)/(Fluorescence intensity in the absence of antigen) | | | | | |

### Example 10

### (Measurement of morphines, methamphetamines, and cocaine using fluoro-labeled Fab complex)

A same-color double-label Fab complex comprising a polypeptide containing an anti-morphine antibody TAMRA-labeled light chain variable region (VL; SEQ ID NO: 23) and an antibody light chain constant domain (Cκ; SEQ ID NO: 4) and a polypeptide containing a TAMRA-labeled anti-morphine antibody heavy chain variable region (VH; SEQ ID NO: 24) and an antibody heavy chain constant domain (CH₁; SEQ ID NO: 6) was synthesized by the method shown in Example 1. Similarly, a same-color double-label Fab complex comprising a polypeptide containing an anti-methamphetamine antibody TAMRA-labeled light chain variable region (VL; SEQ ID NO: 25) and an antibody light chain constant domain (Cκ; SEQ ID NO: 4) and a polypeptide containing a TAMRA-labeled anti-methamphetamine antibody heavy chain variable region (VH; SEQ ID NO: 26) and an antibody heavy chain constant domain (CH₁; SEQ ID NO: 6) was synthesized. Moreover, a same-color double-label Fab complex comprising a polypeptide containing an anti-cocaine antibody TAMRA-labeled light chain variable region (VL; SEQ ID NO: 27) and an antibody light chain constant domain (Cκ; SEQ ID NO: 4) and a polypeptide containing a TAMRA-labeled anti-cocaine antibody heavy chain variable region (VH; SEQ ID NO: 28) and an antibody heavy chain constant domain (CH₁; SEQ ID NO: 6) was synthesized. Furthermore, according to Example 1, a single-chain antibody (scFv) was prepared by linking the TAMRA-labeled anti-morphine antibody VL and VH using a linker (GGGGSGGGGSGGGGS) and a single-chain antibody (scFv) was prepared by linking the anti-methamphetamine antibody VH and VL using a linker (GGGGSGGGGSGGGGS). The thus constructed TAMRA double-label Fab complex and the TAMRA-labeled scFv were reacted with various morphines, methamphetamines, cocaine, and ketamine, and then fluorescence intensity was measured following the method of Example 3 or Example 1. The results are shown in Table 4. The three types of fluoro-labeled Fab complex specifically recognized each antigen. Furthermore, when the fluoro-labeled morphine Fab complex was compared with scFv, and when the fluoro-labeled methamphetamine Fab complex was compared with scFv for fluorescence enhancement (fluorescence intensity ratio), fluorescence enhancement was always higher in the Fab complexes and the dynamic range was significantly increased.

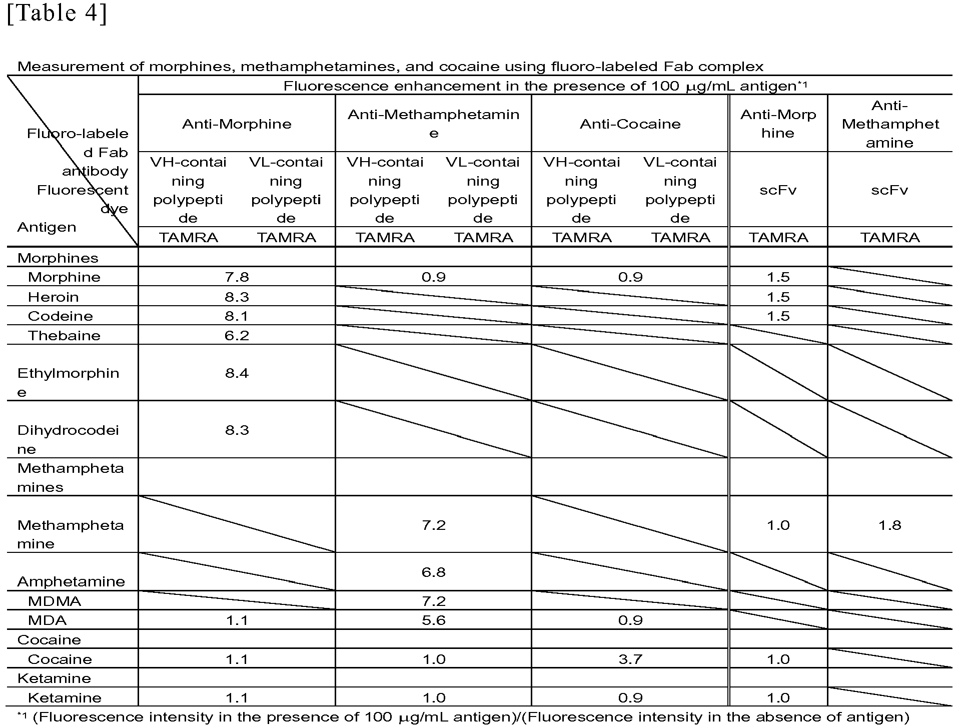

### Example 11

### (Measurement of cannabinoid component THC and ketamine using fluoro-labeled Fab complex)

A same-color double-label Fab complex comprising a polypeptide containing an anti-THC (cannabinoid component) antibody TAMRA-labeled light chain variable region (VL; SEQ ID NO: 29) and an antibody light chain constant domain (Cκ; SEQ ID NO: 4) and a polypeptide containing a TAMRA-labeled anti-THC (cannabinoid component) antibody heavy chain variable region (VH; SEQ ID NO: 30) and an antibody heavy chain constant domain (CH₁; SEQ ID NO: 6) was synthesized by the method described in Example 1. Similarly, a same-color double-label Fab complex comprising a polypeptide containing an anti-ketamine antibody TAMRA-labeled light chain variable region (VL; SEQ ID NO: 31) and an antibody light chain constant domain (Cκ; SEQ ID NO: 4) and a polypeptide containing a TAMRA-labeled anti-ketamine antibody heavy chain variable region (VH; SEQ ID NO: 32) and an antibody heavy chain constant domain (CH₁; SEQ ID NO: 6) was synthesized. Subsequently, each complex was reacted with antigenic THC (100 µg/mL) or ketamine (1.0 mg/mL), and fluorescence intensity was measured following the method of Example 4. As shown in Table 5, measurement was successfully performed with fluorescence intensity ratios (indicating fluorescence enhancement) of 1.3 and 2.0.

**[Table 5]**

| Measurement of cannabinoid component THC and ketamine using fluoro-labeled Fab complex | | | | | |
|---|---|---|---|---|---|
| Fluoro-labeled Fab antibody | Antigen | Labeling fluorescent dye | | Measurement | Fluorescence enhancement |
| | | VH-containing polypeptide | VL-containing polypeptide | Ex/Em | |
| Anti-THC | THC | TAMRA | TAMRA | 530/580 | 1.3^{*1} |
| Anti-Ketamine | Ketamine | TAMRA | TAMRA | 530/580 | 2.0^{*2} |

| | | | | | |
|---|---|---|---|---|---|
| ^{*1} (Fluorescence intensity in the presence of 100 µg/mL THC)/(Fluorescence intensity in the absence of antigen) ^{*2} (Fluorescence intensity in the presence of 1.0 mg/mL Ketamine)/(Fluorescence intensity in the absence of antigen) | | | | | |

### Industrial Applicability

The present invention can be usefully used in the fields of specimen analyses, drug tests, and portable specimen analysis kits, for example.

## Claims

1. A kit for measuring and/or detecting the concentration of an antigen, which is **characterized by** enabling the measurement of the concentration of an antigen or the visualization of an antigen using a positive correlation between the concentration of an antigen and the fluorescence intensity of a fluorescent dye in a liquid phase, as an indicator, wherein,
either or both a polypeptide containing an antibody light chain variable region and a polypeptide containing an antibody heavy chain variable region are labeled with a fluorescent dye, and
the kit is provided with a complex comprising the polypeptide containing the antibody light chain variable region and the polypeptide containing the antibody heavy chain variable region.

2. The kit for measuring and/or detecting the concentration of an antigen according to claim 1, which is **characterized in that** the polypeptide containing the antibody light chain variable region and the polypeptide containing the antibody heavy chain variable region are each labeled with the same fluorescent dye.

3. The kit for measuring and/or detecting the concentration of an antigen according to claim 1, which is **characterized in that** the polypeptide containing the antibody light chain variable region and the polypeptide containing the antibody heavy chain variable region are each labeled with a different type of fluorescent dye.

4. The kit for measuring and/or detecting the concentration of an antigen according to claim 1, which is **characterized in that** either the polypeptide containing the antibody light chain variable region or the polypeptide containing the antibody heavy chain variable region is labeled with a fluorescent dye and the other is labeled with a quencher for quenching the fluorescent dye.

5. The kit for measuring and/or detecting the concentration of an antigen according to claim 1, which is **characterized in that** either the polypeptide containing the antibody light chain variable region or the polypeptide containing the antibody heavy chain variable region is labeled with a fluorescent dye.

6. The kit for measuring and/or detecting the concentration of an antigen according to any one of claims 1 to 5, which is **characterized in that** a complex comprising the polypeptide containing the antibody light chain variable region and the polypeptide containing the antibody heavy chain variable region is a Fab (Fragment, antigen binding).

7. The kit for measuring and/or detecting the concentration of an antigen according to any one of claims 1 to 6, which is **characterized in that** the fluorescent dye is selected from a rhodamine-based fluorescent dye and an oxazine-based fluorescent dye.

8. The kit for measuring and/or detecting the concentration of an antigen according to claim 7, which is **characterized in that** the fluorescent dye is selected from carboxy rhodamine 110, carboxytetramethyl rhodamine, and ATTO 655 (trade name).

9. The kit for measuring and/or detecting the concentration of an antigen according to any one of claims 4 to 8, which is **characterized in that** the quencher is 7-nitrobenzofurazan (NBD).

10. A method for measuring and/or detecting the concentration of an antigen, which is **characterized by** comprising the following steps (a) to (c) in sequence,
(a) a step of bringing a complex into contact with an antigen in a specimen for measurement, wherein
the complex comprises a polypeptide containing an antibody light chain variable region and a polypeptide containing an antibody heavy chain variable region, in which either or both polypeptides are labeled with a fluorescent dye;
(b) a step of detecting the fluorescence of the fluorescent dye, or measuring the fluorescence intensity of the fluorescent dye; and
(c) a step of calculating the amount of the antigen contained in a sample or visualizing the antigen, using a positive correlation between the concentration of the antigen and the fluorescence intensity of the fluorescent dye as an indicator.

11. The method for measuring and/or detecting the concentration of an antigen according to claim 10, which is **characterized in that** the polypeptide containing the antibody light chain variable region and the polypeptide containing the antibody heavy chain variable region are each labeled with the same fluorescent dye.

12. The method for measuring and/or detecting the concentration of an antigen according to claim 10, which is **characterized in that** the polypeptide containing the antibody light chain variable region and the polypeptide containing the antibody heavy chain variable region are each labeled with a different type of fluorescent dye.

13. The method for measuring and/or detecting the concentration of an antigen according to claim 10, which is **characterized in that** either the polypeptide containing the antibody light chain variable region or the polypeptide containing the antibody heavy chain variable region is labeled with a fluorescent dye, and the other is labeled with a quencher for quenching the fluorescent dye.

14. The method for measuring and/or detecting the concentration of an antigen according to claim 10, which is **characterized in that** either the polypeptide containing the antibody light chain variable region or the polypeptide containing the antibody heavy chain variable region is labeled with a fluorescent dye.

15. The method for measuring and/or detecting the concentration of an antigen according to any one of claims 10 to 14, which is **characterized in that** the complex comprising the polypeptide that contains the antibody light chain variable region and the polypeptide that contains the antibody heavy chain variable region is a Fab (Fragment, antigen binding).

16. The method for measuring and/or detecting the concentration of an antigen according to any one of claims 10 to 15, which is **characterized in that** the antigen is a low molecular weight compound.

17. The method for measuring and/or detecting the concentration of an antigen according to any one of claims 10 to 15, which is **characterized in that** the antigen is human osteocalcin, bisphenol A, serum albumin, clenbuterol, ractopamine, cotinine, influenza A virus hemagglutinin, a morphine, a methamphetamine, cocaine, tetrahydrocannabinol, or ketamine.


SEQUENCE LISTING

<110>  Ushio Denki Kabushiki Kaisha

<120>  Fluoroimmunoassay using fluorescence labeled complex composed of VH
and VL containing polypeptides

<130>  08227

<150>  JP 2011-241402

<151>  2011-11-02

<160>  32

<170>  PatentIn version 3.1

<210>  1
<211>  13
<212>  PRT
<213>  Artificial

<220>
<223>  Inventor: Ueda, Hiroshi; Abe, Ryoji; Takagi, Hiroaki

<220>
<223>  ProX tag

<400>  1

Met Ser Lys Gln Ile Glu Val Asn Phe Ser Asn Glu Thr
1               5                   10


<210>  2
<211>  13
<212>  PRT
<213>  Artificial

<220>
<223>  ProX tag containing a fluorescent amino acid

<220>
<221>  MISC_FEATURE
<222>  (9)..(9)
<223>  Xaa is a fluorescent amino acid.


<400>  2

Met Ser Lys Gln Ile Glu Val Asn Xaa Ser Asn Glu Thr
1               5                   10


<210>  3
<211>  113
<212>  PRT
<213>  Artificial

<220>
<223>  anti-BGP antibody VH

<400>  3

Asp Ile Glu Leu Thr Gln Ser Pro Leu Ser Leu Pro Val Ser Leu Gly
1               5                   10                  15

Asp Gln Ala Ser Ile Ser Cys Thr Ser Ser Gln Ser Leu Leu His Ser
            20                  25                  30

Asn Gly Asp Thr Tyr Leu His Trp Tyr Leu Gln Lys Pro Gly Gln Ser
        35                  40                  45

Pro Lys Leu Leu Ile Tyr Thr Leu Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Ala Asp Leu Gly Ile Tyr Phe Cys Ser Gln Thr
                85                  90                  95

Thr His Val Pro Tyr Thr Phe Gly Gly Gly Thr Lys Leu Glu Ile Lys
            100                 105                 110

Arg

<210>  4
<211>  106
<212>  PRT
<213>  Artificial

<220>
<223>  peptide Ck

<400>  4

Ala Asp Ala Ala Pro Thr Val Ser Ile Phe Pro Pro Ser Ser Glu Gln
1               5                   10                  15

Leu Thr Ser Gly Gly Ala Ser Val Val Cys Phe Leu Asn Asn Phe Tyr
            20                  25                  30

Pro Lys Asp Ile Asn Val Lys Trp Lys Ile Asp Gly Ser Glu Arg Gln
        35                  40                  45

Asn Gly Val Leu Asn Ser Trp Thr Asp Gln Asp Ser Lys Asp Ser Thr
    50                  55                  60

Tyr Ser Met Ser Ser Thr Leu Thr Leu Thr Lys Asp Glu Tyr Glu Arg
65                  70                  75                  80

His Asn Ser Tyr Thr Cys Glu Ala Thr His Lys Thr Ser Thr Ser Pro
                85                  90                  95

Ile Val Lys Ser Phe Asn Arg Asn Glu Cys
            100                 105

<210>  5
<211>  116
<212>  PRT
<213>  Artificial

<220>
<223>  anti-BGP antibody VL

<400>  5

Gln Val Lys Leu Gln Gln Ser Gly Ala Glu Phe Val Lys Ala Gly Ala
1               5                   10                  15

Ser Val Lys Leu Ser Cys Lys Thr Ser Gly Tyr Thr Phe Asn Asn Tyr
            20                  25                  30

Trp Ile His Trp Val Lys Gln Ser Pro Gly Gln Gly Leu Glu Trp Ile
        35                  40                  45

Gly Glu Ile Asp Pro Ser Asp Gly Tyr Ser Asn Tyr Asn Gln Lys Phe
    50                  55                  60

Lys Gly Lys Ala Thr Leu Thr Val Asp Lys Ser Ser Ser Thr Ala Tyr
65                  70                  75                  80

Met His Leu Asn Ser Leu Thr Ser Glu Asp Ser Ala Val Tyr Tyr Cys
                85                  90                  95

Thr Ser Ser Thr Ser Val Gly Gly Ser Trp Gly Gln Gly Thr Thr Val
            100                 105                 110

Thr Val Ser Ser
        115

<210>  6
<211>  102
<212>  PRT
<213>  Artificial

<220>
<223>  peptide CH1

<400>  6

Ala Lys Thr Thr Pro Pro Ser Val Tyr Pro Leu Ala Pro Gly Ser Ala
1               5                   10                  15

Ala Gln Thr Asn Ser Met Val Thr Leu Gly Cys Leu Val Lys Gly Tyr
            20                  25                  30

Phe Pro Glu Pro Val Thr Val Thr Trp Asn Ser Gly Ser Leu Ser Ser
        35                  40                  45

Gly Val His Thr Phe Pro Ala Val Leu Gln Ser Asp Leu Tyr Thr Leu
    50                  55                  60

Ser Ser Ser Val Thr Val Pro Ser Ser Thr Trp Pro Ser Glu Thr Val
65                  70                  75                  80

Thr Cys Asn Val Ala His Pro Ala Ser Ser Thr Lys Val Asp Lys Lys
                85                  90                  95

Ile Val Pro Arg Asp Cys
            100

<210> 7
<211> 111
<212> PRT
<213> Artificial

<220>
<223> anti-bisphenol A antibody VL

<400> 7

Asp Ile Val Leu Thr Gln Ser Pro Ala Ser Leu Ala Val Ser Leu Gly
1               5                   10                  15

Gln Arg Ala Thr Ile Ser Cys Arg Ala Ser Gln Ser Val Ser Thr Ser
            20                  25                  30

Thr Tyr Ser Tyr Leu His Trp Tyr Gln Gln Arg Pro Gly Gln Pro Pro
        35                  40                  45

Lys Leu Ile Lys Tyr Val Ser Asn Leu Glu Ser Gly Val Pro Ala Arg
    50                  55                  60

Phe Ser Gly Ser Gly Ser Gly Thr Asp Phe Thr Leu Asn Ile His Pro
65                  70                  75                  80

Val Glu Glu Glu Asp Thr Ala Thr Tyr Tyr Cys Gln His Ser Trp Glu
                85                  90                  95

Ile Pro Pro Thr Phe Gly Gly Gly Thr Lys Leu Glu Ile Lys Arg
            100                 105                 110

<210>  8
<211>  20
<212>  PRT
<213>  Artificial

<220>
<223>  spacer GGGS5

<400>  8

Gly Gly Gly Ser Gly Gly Gly Ser Gly Gly Gly Ser Gly Gly Gly Ser
1               5                   10                  15

Gly Gly Gly Ser
            20

<210>  9
<211>  119
<212>  PRT
<213>  Artificial

<220>
<223>  anti-bisphenol A antibody VH

<400>  9

Asp Val Gln Leu Gln Glu Ser Gly Pro Gly Leu Val Lys Pro Ser Gln
1               5                   10                  15

Ser Leu Ser Leu Thr Cys Ser Val Thr Gly Tyr Ser Ile Thr Ser Gly
            20                  25                  30

Tyr Tyr Trp Asn Trp Ile Arg Gln Phe Pro Gly Asn Lys Leu Glu Trp
        35                  40                  45

Met Gly Tyr Ile Arg Tyr Asp Gly Ser Asn Asn Tyr Asn Pro Ser Leu
    50                  55                  60

Lys Asn Arg Ile Ser Ile Thr Arg Asp Thr Ser Lys Asn Gln Phe Phe
65                  70                  75                  80

Leu Lys Leu Asn Ser Val Thr Pro Glu Asp Thr Ala Thr Tyr Tyr Cys
                85                  90                  95

Ala Arg Val Leu Gly Arg Gly Tyr Gly Leu Asp Tyr Trp Gly Gln Gly
            100                 105                 110

Thr Ser Val Thr Val Ser Ser
        115

<210>  10
<211>  108
<212>  PRT

<213>  Artificial

<220>
<223>  anti-SA antibody VL

<400>  10

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Ser Tyr
            20                  25                  30

Leu Asn Trp Tyr Gln Gln Lys Pro Gly Lys Ala Pro Lys Leu Leu Ile
        35                  40                  45

Tyr Arg Ala Ser Leu Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Arg Asp Arg Pro Ala
                85                  90                  95

Thr Phe Gly Gln Gly Thr Lys Val Glu Ile Lys Arg
            100                 105

<210>  11
<211>  8
<212>  PRT
<213>  Artificial

<220>
<223>  spacer GGGS2

<400>  11

Gly Gly Gly Ser Gly Gly Gly Ser
1               5

<210>  12
<211>  116
<212>  PRT
<213>  Artificial

<220>
<223>  anti-SA antibody VH

<400>  12

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser His Ile Ser Pro Tyr Gly Ala Asn Thr Arg Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Leu Arg Ala Phe Asp Tyr Trp Gly Gln Gly Thr Leu Val
            100                 105                 110

Thr Val Ser Ser
        115

<210>  13
<211>  7
<212>  PRT
<213>  Artificial

<220>
<223>  peptide BGP-C7

<400>  13

Arg Arg Phe Tyr Gly Pro Val
1               5

<210>  14
<211>  4
<212>  PRT
<213>  Artificial

<220>
<223>  linker GGGS

<400>  14

Gly Gly Gly Ser
1

<210>  15
<211>  107
<212>  PRT
<213>  Artificial

<220>
<223>  Clen-VL-15

<400>  15

Asp Ile Glu Leu Thr Gln Ser Pro Ala Ile Leu Ser Ala Ser Pro Gly
1               5                   10                  15

Glu Lys Val Thr Met Thr Cys Ser Ala Asn Ser Ser Val Ser Tyr Met
            20                  25                  30

His Trp Tyr Gln Gln Lys Ser Arg Thr Ser Pro Lys Leu Trp Ile Tyr
        35                  40                  45

Asp Thr Ser Lys Leu Ala Ser Gly Val Pro Gly Arg Phe Ser Gly Ser
    50                  55                  60

Gly Ser Gly Asn Ser Tyr Ser Leu Thr Ile Ser Ser Met Glu Ala Glu
65                  70                  75                  80

Asp Val Ala Thr Tyr Tyr Cys Phe Gln Gly Ser Lys Tyr Pro Phe Thr
                85                  90                  95

Phe Gly Ser Gly Thr Arg Leu Glu Ile Lys Arg
            100                 105


<210>  16
<211>  118
<212>  PRT
<213>  Artificial

<220>
<223>  Clen-VH-16

<400>  16

Gln Val Lys Leu Gln Glu Ser Gly Gly Gly Leu Val Lys Pro Arg Gly
1               5                   10                  15

Ser Leu Lys Leu Ser Cys Ala Ala Ser Gly Phe Thr Phe Ser Ser Tyr
            20                  25                  30

Asp Met Ser Trp Val Arg Gln Thr Pro Glu Lys Arg Leu Glu Trp Val
        35                  40                  45

Ala Ser Ile Arg Ser Gly Gly Tyr Tyr Thr Leu Tyr Pro Asp Ser Val
    50                  55                  60

Lys Gly Arg Phe Thr Ile Ser Arg Asp Asn Val Lys Asn Thr Leu Tyr
65                  70                  75                  80

Leu Gln Met Ser Ser Leu Arg Ser Glu Asp Thr Ala Met Tyr Tyr Cys
                85                  90                  95

Ser Arg Asp Gly Asn Asp Glu Tyr Phe Asp Val Trp Gly Ala Gly Thr
            100                 105                 110

Thr Val Thr Val Ser Ser
        115

<210>  17
<211>  113
<212>  PRT
<213>  Artificial

<220>
<223>  Ract-VL-17

<400>  17

Asp Ile Leu Met Thr Gln Thr Pro Leu Ser Leu Pro Val Ser Leu Gly
1               5                   10                  15

Asp Gln Ala Ser Ile Ser Cys Arg Ser Ser Gln Asn Ile Val His Ser
            20                  25                  30

Asp Gly Asn Thr Tyr Leu Glu Trp Tyr Leu Gln Lys Pro Gly Gln Ser
        35                  40                  45

Pro Lys Leu Leu Ile Ser Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Ile Tyr Tyr Cys Phe Gln Gly
                85                  90                  95

Ser His Val Pro Trp Thr Phe Gly Gly Gly Thr Lys Leu Glu Ile Lys
            100                 105                 110

Arg

<210>  18
<211>  123
<212>  PRT
<213>  Artificial

<220>
<223>  Ract-VH-18

<400>  18

Asp Val Gln Leu Val Glu Ser Gly Pro Gly Leu Val Lys Pro Ser Gln

1               5                   10                  15

Ser Leu Ser Leu Thr Cys Thr Val Thr Gly Tyr Ser Ile Thr Ser Asp
            20                  25                  30

Tyr Ala Trp Asn Trp Ile Arg Gln Phe Pro Gly Asn Lys Leu Glu Trp
        35                  40                  45

Met Gly Tyr Ile Phe Tyr Ser Gly Ser Thr Ser Tyr Asn Pro Ser Leu
    50                  55                  60

Lys Ser Arg Ile Ser Ile Thr Arg Asp Thr Ser Lys Asn Gln Phe Phe
65                  70                  75                  80

Leu Gln Leu Asn Ser Val Thr Thr Glu Asp Thr Ala Thr Tyr Phe Cys
                85                  90                  95

Ala Arg Leu Ala Tyr Tyr Tyr Asp Tyr Asp Gly Tyr Ala Met Asp Tyr
            100                 105                 110

Trp Gly Gln Gly Thr Ser Val Thr Val Ser Ser
        115                 120

<210>  19
<211>  112
<212>  PRT
<213>  Artificial

<220>
<223>  Coti-VL-19

<400>  19

Glu Leu Asp Leu Thr Gln Thr Pro Ser Pro Val Ser Ala Ala Val Gly
1               5                   10                  15

Asp Thr Val Thr Ile Asn Cys Gln Ser Ser Gln Ser Val Tyr Ser Ala
            20                  25                  30

Lys Leu Ser Trp Tyr Gln Gln Lys Pro Gly Gln Pro Pro Lys Leu Leu
        35                  40                  45

Ile Tyr Tyr Gly Ser Thr Leu Ala Ser Gly Val Pro Ser Arg Phe Lys
    50                  55                  60

Gly Ser Gly Ser Gly Thr Gln Phe Ser Leu Thr Ile Ser Asp Val Gln
65                  70                  75                  80

Cys Ala Asp Ala Ala Thr Tyr Tyr Cys Gln Gly Thr Tyr Tyr Gly Pro
                85                  90                  95

Asp Trp Tyr Phe Ala Phe Gly Gly Gly Thr Glu Val Val Val Lys Arg
            100                 105                 110

<210> 20
<211> 116
<212> PRT
<213> Artificial

<220>
<223> Coti-VH-20

<400> 20

Gln Gln Gln Leu Val Glu Ser Gly Gly Arg Leu Val Thr Pro Gly Gly
1               5                   10                  15

Ser Leu Thr Leu Thr Cys Thr Ala Ser Gly Phe Ser Leu Asn Asn Tyr
            20                  25                  30

Trp Met Ser Trp Val Arg Gln Ala Pro Gly Lys Gly Leu Glu Trp Ile
        35                  40                  45

Gly Asp Ile His Gly Asn Arg Gly Phe Asn Tyr His Ala Ser Trp Ala
    50                  55                  60

Lys Gly Arg Phe Thr Val Ser Arg Thr Ser Thr Thr Val Asp Leu Arg
65                  70                  75                  80

Met Thr Ser Leu Thr Thr Glu Asp Thr Ala Ile Tyr Phe Cys Ala Arg
                85                  90                  95

Ala Asp Asp Ser Gly Ser His Asp Ile Trp Gly Pro Gly Thr Leu Val
            100                 105                 110

Thr Val Ser Ser
        115

<210> 21
<211> 112
<212> PRT
<213> Artificial

<220>
<223> Infl-VL-21

<400> 21

Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Val Thr Phe Asn

20                  25                  30

Tyr Lys Asn Tyr Leu Ala Trp Tyr Gln Gln Lys Pro Gly Gln Pro Pro
        35                  40                  45

Lys Leu Leu Ile Tyr Trp Ala Ser Thr Arg Glu Ser Gly Val Pro Asp
    50                  55                  60

Arg Phe Ser Gly Ser Gly Ser Gly Thr Asp Phe Thr Leu Thr Ile Ser
65                  70                  75                  80

Ser Leu Gln Ala Glu Asp Val Ala Val Tyr Tyr Cys Gln Gln His Tyr
                85                  90                  95

Arg Thr Pro Pro Thr Phe Gly Gln Gly Thr Lys Val Glu Ile Lys Arg
            100                 105                 110

<210>  22
<211>  129
<212>  PRT
<213>  Artificial

<220>
<223>  Infl-VH-22

<400>  22

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Thr Tyr
            20                  25                  30

Ala Met His Trp Val Arg Gln Ala Pro Gly Lys Gly Leu Glu Trp Val
        35                  40                  45

Ala Val Ile Ser Tyr Asp Ala Asn Tyr Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Ser Gln Leu Arg Ser Leu Leu Tyr Phe Glu Trp Leu Ser
            100                 105                 110

Gln Gly Tyr Phe Asp Tyr Trp Gly Gln Gly Thr Leu Val Thr Val Ser
        115                 120                 125

Ser

<210>  23
<211>  109
<212>  PRT
<213>  Artificial

<220>
<223>  Morp-VL-23

<400>  23

Gln Ala Val Val Thr Gln Glu Ser Ala Leu Thr Thr Ser Pro Gly Glu
1               5                   10                  15

Thr Val Thr Leu Thr Cys Arg Ser Ser Thr Gly Ala Val Thr Thr Ser
            20                  25                  30

Asn Tyr Ala Asn Trp Val Gln Glu Lys Pro Asp His Leu Phe Thr Gly
        35                  40                  45

Leu Ile Gly Gly Thr Asn Asn Arg Ala Pro Gly Val Pro Ala Arg Phe
    50                  55                  60

Ser Gly Ser Leu Ile Gly Asp Lys Ala Ala Leu Thr Ile Thr Gly Ala
65                  70                  75                  80

Gln Thr Glu Asp Glu Ala Ile Tyr Phe Cys Val Leu Trp Tyr Ser Asn
                85                  90                  95

His Leu Val Phe Gly Gly Gly Thr Lys Leu Thr Val Leu
            100                 105

<210>  24
<211>  118
<212>  PRT
<213>  Artificial

<220>
<223>  Morp-VH-24

<400>  24

Gln Val Gln Leu Gln Gln Ser Gly Pro Glu Leu Met Lys Pro Gly Ala
1               5                   10                  15

Ser Val Lys Ile Ser Cys Lys Ala Thr Gly Tyr Thr Phe Ser Ser His
            20                  25                  30

Trp Ile Glu Trp Val Lys Gln Arg Pro Gly His Gly Leu Glu Trp Ile

35                  40                  45

Gly Glu Ile Leu Pro Gly Ser Gly Ser Thr Lys Tyr Asn Glu Lys Phe
    50                  55                  60

Lys Gly Lys Ala Thr Phe Thr Ala Asp Thr Ser Ser Asn Thr Val Tyr
65                  70                  75                  80

Met Gln Leu Ser Ser Leu Thr Ser Glu Asp Ser Ala Val Tyr His Cys
                85                  90                  95

Ala Arg Trp Ser Gln Val His Val Met Asp Tyr Trp Gly Gln Gly Thr
            100                 105                 110

Ser Val Thr Val Ser Ser
        115

<210>  25
<211>  109
<212>  PRT
<213>  Artificial

<220>
<223>  Meth-VL-25

<400>  25

Asp Ile Val Met Thr Gln Ser Pro Ala Phe Met Ser Ala Ser Pro Gly
1               5                   10                  15

Glu Lys Val Thr Leu Thr Cys Ser Ala Ser Ser Ser Val Ser Ser Thr
            20                  25                  30

Phe Leu Tyr Trp Tyr Gln Gln Lys Pro Gly Ser Ser Pro Lys Leu Trp
        35                  40                  45

Ile Tyr Ser Thr Ser Asn Leu Ala Ser Gly Val Pro Ser Arg Phe Ser
    50                  55                  60

Gly Ser Gly Ser Gly Thr Ser Tyr Ser Leu Thr Ile Asn Ser Met Glu
65                  70                  75                  80

Ala Glu Asp Ala Ala Ser Tyr Phe Cys His Gln Trp Ser Asn Tyr Pro
                85                  90                  95

Phe Thr Phe Gly Ser Gly Thr Lys Leu Glu Ile Lys Arg
            100                 105

<210>  26
<211>  120

<212>  PRT
<213>  Artificial

<220>
<223>  Meth-VH-26

<400>  26

Gln Val Gln Leu Gln Gln Ser Gly Pro Glu Leu Val Lys Pro Gly Ala
1               5                   10                  15

Ser Val Lys Val Ser Cys Lys Ala Ser Gly Tyr Pro Ser Thr Arg Phe
            20                  25                  30

Tyr Ile Tyr Trp Val Lys Gln Ser His Gly Lys Ser Leu Glu Trp Ile
        35                  40                  45

Gly Asn Ile Asp Pro Tyr Asn Gly Gly Thr Thr Tyr Asn Gln Lys Phe
    50                  55                  60

Lys Gly Lys Ala Thr Leu Thr Ile Asp Lys Ser Ser Thr Thr Ala Tyr
65                  70                  75                  80

Val His Leu Asn Ser Leu Thr Ser Glu Asp Ser Ala Val Tyr Tyr Cys
                85                  90                  95

Ala Gly Phe His Tyr Ser Gly Gln Leu Asp Thr Asp Val Trp Gly Ala
            100                 105                 110

Gly Thr Lys Val Thr Val Ser Ser
        115                 120


<210>  27
<211>  113
<212>  PRT
<213>  Artificial

<220>
<223>  Coca-VL-27

<400>  27

Asp Ile Val Met Thr Gln Ala Ala Pro Ser Val Pro Val Thr Pro Gly
1               5                   10                  15

Glu Ser Val Ser Ile Ser Cys Arg Ser Ser Lys Ser Leu Leu His Ser
            20                  25                  30

Asn Gly Tyr Thr Tyr Leu His Trp Phe Leu Gln Arg Pro Gly Gln Ser
        35                  40                  45

Pro Gln Leu Leu Ile Tyr Arg Val Ser Asn Leu Ala Ser Gly Val Pro

50                  55                  60

Asp Arg Phe Ser Gly Ser Gly Ser Gly Thr Ala Phe Thr Leu Arg Phe
65                  70                  75                  80

Ser Arg Val Glu Ala Glu Asp Val Gly Val Tyr Tyr Cys Met Gln His
                85                  90                  95

Leu Glu Tyr Pro Phe Thr Phe Gly Ser Gly Thr Lys Leu Glu Ile Lys
            100                 105                 110

Arg

<210>  28
<211>  121
<212>  PRT
<213>  Artificial

<220>
<223>  Coca-VH-28

<400>  28

Asp Val Thr Leu Gln Glu Ser Gly Gly Gly Leu Val Gln Pro Gly Gly
1               5                   10                  15

Ser Met Lys Leu Ser Cys Ala Ala Ser Gly Phe Thr Phe Ser Asp Ala
            20                  25                  30

Trp Val Asp Trp Val Arg Gln Ser Pro Gly Lys Gly Leu Glu Trp Val
        35                  40                  45

Ala Glu Ile Arg Asn Lys Ala Asn Asn His Ala Thr Lys Tyr Thr Glu
    50                  55                  60

Ser Val Lys Gly Arg Phe Thr Ile Ser Arg Asp Asp Ser Lys Ser Ser
65                  70                  75                  80

Val Tyr Leu Gln Met Asn Ser Leu Arg Ala Glu Asp Thr Gly Ile Tyr
                85                  90                  95

Tyr Cys Thr Ser Val Pro Gln Leu Gly Arg Gly Phe Ala Tyr Trp Gly
            100                 105                 110

Gln Gly Thr Leu Val Thr Val Ser Ser
        115                 120

<210>  29
<211>  109

<212>  PRT
<213>  Artificial

<220>
<223>  THC-VL-29

<400>  29

Asp Ile Val Leu Thr Gln Ser Pro Thr Thr Met Ala Ala Ser Pro Gly
1               5                   10                  15

Glu Lys Ile Thr Ile Thr Cys Ser Ala Ser Ser Ser Ile Ser Ser Asn
            20                  25                  30

Tyr Leu His Trp Tyr Gln Gln Lys Pro Gly Phe Ser Pro Lys Leu Leu
        35                  40                  45

Ile Tyr Arg Thr Ser Asn Leu Ala Ser Gly Val Pro Ala Arg Phe Ser
    50                  55                  60

Gly Ser Gly Ser Gly Thr Ser Tyr Ser Leu Thr Ile Gly Thr Met Glu
65                  70                  75                  80

Ala Glu Asp Val Ala Thr Tyr Tyr Cys Gln Gln Gly Ser Ser Ile Pro
                85                  90                  95

Leu Thr Phe Gly Ala Gly Thr Lys Leu Glu Leu Lys Arg
            100                 105


<210>  30
<211>  117
<212>  PRT
<213>  Artificial

<220>
<223>  THC-VH-30

<400>  30

Glu Val Lys Leu Val Glu Ser Gly Gly Gly Leu Val Lys Pro Gly Gly
1               5                   10                  15

Ser Leu Lys Leu Ser Cys Ala Ala Ser Gly Phe Thr Phe Asn Asn Tyr
            20                  25                  30

Val Met Val Trp Leu Arg Gln Thr Pro Glu Lys Arg Leu Glu Trp Val
        35                  40                  45

Ala Ser Ile Ser Arg Gly Gly Ser Thr Tyr Tyr Pro Asp Ser Val Lys
    50                  55                  60

Gly Arg Phe Thr Ile Ser Arg Asp Asn Ala Arg Asn Ile Leu Tyr Leu

65                  70                  75                  80

Gln Met Ser Ser Leu Arg Ser Glu Asp Thr Ala Met Tyr Tyr Cys Val
                85                  90                  95

Arg Gly Thr Thr Ile Val Ala Gly Asp Val Trp Gly Ala Gly Thr Thr
            100                 105                 110

Val Thr Val Ser Ser
        115

<210>  31
<211>  111
<212>  PRT
<213>  Artificial

<220>
<223>  Keta-VL-31

<400>  31

Gln Ala Val Val Thr Gln Glu Ser Ala Leu Thr Thr Ser Pro Gly Glu
1               5                   10                  15

Thr Val Thr Leu Thr Cys Arg Ser Ser Thr Gly Ala Val Thr Thr Ser
            20                  25                  30

Asn Tyr Ala Asn Trp Val Gln Glu Lys Pro Asp His Leu Phe Thr Gly
        35                  40                  45

Leu Ile Gly Gly Thr Asn Asn Arg Ala Pro Gly Val Pro Ala Arg Phe
    50                  55                  60

Ser Gly Ser Leu Ile Gly Asp Lys Ala Ala Leu Thr Ile Thr Gly Ala
65                  70                  75                  80

Gln Thr Glu Asp Glu Ala Ile Tyr Phe Cys Ser Leu Trp Tyr Ser Asn
                85                  90                  95

His Leu Val Phe Gly Gly Gly Thr Lys Leu Thr Val Leu Gly Gln
            100                 105                 110

<210>  32
<211>  118
<212>  PRT
<213>  Artificial

<220>
<223>  Keta-VH-32

<400>  32

Gln Val Gln Leu Lys Glu Ser Gly Pro Gly Leu Val Ala Pro Ser Gln
1               5                   10                  15

Ser Leu Ser Ile Thr Cys Thr Val Ser Gly Phe Ala Leu Thr Gly Tyr
            20                  25                  30

Gly Val Asn Trp Val Arg Gln Pro Pro Gly Lys Gly Leu Glu Trp Leu
        35                  40                  45

Gly Met Ile Trp Gly Asp Gly Asn Thr Asp Tyr Asn Ser Ala Leu Lys
    50                  55                  60

Ser Arg Leu Ser Ile Ser Lys Asp Asn Ser Lys Ser Gln Val Phe Leu
65                  70                  75                  80

Lys Met Asn Ser Leu Gln Thr Asp Asp Thr Ala Arg Tyr Tyr Cys Ala
                85                  90                  95

Arg Gly Asn Gly Tyr Phe Tyr Val Leu Asp Tyr Trp Gly Gln Gly Thr
            100                 105                 110

Ser Val Thr Val Ser Ser
        115